# EUROPEAN PATENT APPLICATION

(11) **EP 4 748 423 A1**
(43) Date of publication of application: **27.05.2026**
(21) Application number: 24844178.4
(22) Date of filing: 18.01.2024
(51) Int. Cl.: A61M 16/16, A61M 16/14, A61M 16/10

(54) **HUMIDIFICATION TANK OF VENTILATOR**

(30) Priority: 21.07.2023 CN 202310905161; 21.07.2023 CN 202310910704
(71) Applicant: Jiangsu Yuyue Medical Equipment & Supply Co., Ltd., Zhenjiang, Jiangsu 212300 (CN); Nanjing Yuyue Software Technology Co., Ltd., Nanjing, Jiangsu 210023 (CN); Suzhou Yuyue Medical Technology Co., Ltd, Suzhou, Jiangsu 215600 (CN)
(72) Inventor: LIU, Yun, Zhenjiang, Jiangsu 212300 (CN); GUO, Jianming, Zhenjiang, Jiangsu 212300 (CN); ZHU, Jing, Zhenjiang, Jiangsu 212300 (CN); ZHU, Tingting, Zhenjiang, Jiangsu 212300 (CN); ZHAO, Shuai, Zhenjiang, Jiangsu 212300 (CN); WU, Qun, Zhenjiang, Jiangsu 212300 (CN)
(74) Representative: Huang, Liwei
(86) International application number: PCT/CN2024/073126
(87) International publication number: WO 2025/020478

(57) **Abstract**

The present invention discloses a humidification tank for a ventilator, comprising a tank body and an internal chamber. The internal chamber is configured to contain a humidification liquid and to communicate with an external air path via an airflow inlet and an airflow outlet of the tank body; and an insertion end of the tank body is configured to be inserted into the ventilator. The humidification tank further comprises an intake airflow guide portion having a first end and a second end. The intake airflow guide portion comprises a bent flow guide pipe having at least two bends, such that the first end and the second end are located on two opposite sides; or, the intake airflow guide portion comprises a flow guide pipe which is bent and extends, and the flow guide pipe comprises a first flow channel and a second flow channel. When the humidification tank is rotated from an operating orientation in any direction by an angle of 90° or less, the humidification liquid cannot enter the first flow channel, so that the humidification liquid is prevented from flowing out of the airflow inlet. The bent flow guide pipe enables a flowing path of airflow to be longer and more tortuous, thereby reducing noise generated in the flowing process of airflow, and also lengthening a path for the liquid to flow back to the airflow inlet and thus reducing the risk of back flow.

## Description

### CROSS-REFERENCE TO RELATED APPLICATIONS

The present invention claims the benefit of priority of Chinese Patent Application No. 202310905161.4 filed with the China National Intellectual Property Administration on July 21, 2023, and titled "HUMIDIFICATION TANK OF VENTILATOR", and Chinese Patent Application No. 202310910704.1 filed with the China National Intellectual Property Administration on July 21, 2023, and titled "HUMIDIFICATION TANK OF VENTILATOR", the entire content of which are incorporated herein by reference.

### TECHNICAL FIELD

The present invention relates to the technical field of medical devices, and specifically to a humidification tank of a ventilator.

### BACKGROUND

Generally, a ventilator is provided with a blower, and achieves air exchange with a user or a patient by means of a respiratory hose connected to the air outlet of the blower and a mask. Humidified air can make the patient or the user feel more comfortable, and is easier to receive. Therefore, a humidification assembly has gradually become one of the indispensable components of a ventilator.

Usually, the humidification assembly is a humidification tank arranged in a ventilator, and is provided inside with an air duct assembly and a humidification liquid. When an airflow sent by a blower enters the interior of the humidification tank through the air duct assembly, it contacts the humidification liquid and gets moistened. Then the moistened air flows out from the outlet of the humidification tank to a user end to assist the user in breathing.

With the increase in user needs, ventilators have gradually entered homes. Especially, small household ventilators are becoming more and more popular. However, due to their small size and light weight, such ventilators feature poor stability, making them easier to tip over. Especially, when a user uses the ventilator during sleep, a turnover might cause the ventilator to tip over and the humidification tank to tip over too. In this case, the humidification liquid tends to flow back into the air duct assembly, and then further flows back from the inlet of the air duct assembly to the blower, making the blower prone to risks such as short-circuiting and damage.

A humidification tank must be designed to ensure that it is capable of containing a sufficient amount of humidification liquid to ensure the humidification effect, and the airflow has a large enough contact area with the humidification liquid.

In addition, when the airflow flows in the air duct assembly, a loud howling sound will be generated, and when the airflow contacts the humidification liquid, it will also cause disturbance to the humidification liquid and generate noise, making the ventilator noisy during use. As many users choose to sleep on ventilators, the excessive noise from the ventilator directly affects their sleep quality, causing a bad user experience.

### SUMMARY

The present invention provides a humidification tank of a ventilator to solve at least one of the above technical problems.

According to a first aspect of the present invention, a humidification tank of a ventilator is provided, and the technical solution includes:
a humidification tank of a ventilator, including a tank body and an internal chamber; the tank body is provided with an airflow inlet and an airflow outlet, and the internal chamber is configured to contain a humidification liquid and be in communication with an external air path via the airflow inlet and the airflow outlet; the tank body includes an insertion end and a graspable end; the insertion end can be inserted into the ventilator unit; the humidification tank further includes an intake airflow guide portion having a first end and a second end; the first end is hermetically connected to the airflow inlet, and the second end is connected to the internal chamber; and the intake airflow guide portion includes a bent flow guide pipe having at least two bends, such that the first end and the second end are located on two opposite sides of the intake airflow guide portion.

The bent flow guide pipe includes a first section, a second section, and a third section, which are connected in sequence; the first section and the third section extend along a first direction; and the second section extends in a direction opposite to the first direction.

The first section, the second section, and the third section are arranged side by side, and the third section is positioned higher than the first section and/or the second section.

The bottom height of the third section gradually decreases along the first direction.

The third section is provided with an anti-backflow rib protruding upward on the bottom wall of the third section.

The change in cross-sectional area between the first section and the second section and/or the change in cross-sectional area between the second section and the third section is less than 10%.

The width of the third section gradually increases along the first direction.

The intake airflow guide portion further includes a flow rectification portion disposed at the second end; an inlet of the flow rectification portion is connected to an outlet of the intake airflow guide portion; an outlet of the flow rectification portion is connected to the internal chamber; and the outlet of the flow rectification portion is positioned lower than the inlet of the flow rectification portion.

The outlet of the flow rectification portion is positioned lower than the first section.

The outlet of the flow rectification portion is configured such that the airflow enters the internal chamber in a direction different from the first direction.

A liquid damper is provided on the lower side of the outlet of the flow rectification portion, and the liquid damper extends toward the first end and to a position below the intake airflow guide portion.

The liquid damper extends obliquely downward to form a flow guide slope on the surface of the liquid damper.

The flow rectification portion is provided with a flow guide rib, which is bent and extends to guide the airflow to flow toward the outlet of the flow rectification portion.

The humidification tank further includes an outtake airflow guide portion having a third end and a fourth end; the third end is in communication with the internal chamber; and the fourth end is hermetically connected to the airflow outlet.

The humidification tank includes a base and a cover configured to be snap-fitted with each other, and the airflow inlet and the airflow outlet are disposed on the cover and extend toward the internal chamber to form an airflow inlet mounting portion and an airflow outlet mounting portion, respectively.

The intake airflow guide portion and the outtake airflow guide portion are configured as an integrated structure, and can be integrally plug-in fitted with the airflow inlet mounting portion and the airflow outlet mounting portion, respectively.

The intake airflow guide portion or the outtake airflow guide portion is provided with a positioning structure, and the cover is provided with a matching structure matched with the positioning structure.

According to a second aspect of this disclosure, a humidification tank of a ventilator is provided, and the technical solution includes:
a humidification tank of a ventilator, including a tank body and an internal chamber; the tank body is provided with an airflow inlet and an airflow outlet, and the internal chamber is configured to contain a humidification liquid and to be in communication with an external air path via the airflow inlet and the airflow outlet; the tank body includes an insertion end and a graspable end; the insertion end is configured to be inserted into the ventilator unit; the humidification tank further includes an intake airflow guide portion having a first end and a second end; the first end is hermetically connected to the airflow inlet, and the second end is connected to the internal chamber; the intake airflow guide portion includes a flow guide pipe which is bent and extends; the flow guide pipe includes a first flow channel and a second flow channel, which are interconnected with each other; and the internal chamber and the flow guide pipe are configured such that when the humidification tank is rotated from an operating orientation in any direction by an angle of 90° or less, the humidification liquid cannot enter the first flow channel, to prevent the humidification liquid from flowing out of the airflow inlet.

The airflow inlet is disposed on the front side of the tank body, both the first flow channel and the second flow channel extend along the front-to-back direction of the tank body, and when the humidification tank rotates forward from the operating orientation by an angle of 90° or less, the second end is higher than the level of the humidification liquid.

The flow guide pipe is provided inside with a partition, which is located between the first flow channel and the second flow channel, and when the humidification tank rotates backward from the operating orientation by an angle of 90° or less, the highest point of the partition is higher than the level of the humidification liquid.

The airflow inlet is disposed on the front side of the tank body, the flow guide pipe is provided inside with a partition, which is located between the first flow channel and the second flow channel, and when the humidification tank is rotated leftward or rightward from the operating orientation by an angle of 90° or less, at least a part of the partition is higher than the level of the humidification liquid.

The intake airflow guide portion further includes a flow rectification portion disposed at the second end thereof; an inlet of the flow rectification portion is connected to an outlet of the intake airflow guide portion; the outlet of the flow rectification portion is connected to the internal chamber; and the position of the outlet of the flow rectification portion is lower than the position of the inlet of the flow rectification portion.

A liquid damper is provided on the lower side of the outlet of the flow rectification portion, and when the humidification tank is in the operating orientation, the lowest end of the liquid damper is higher than the level of the humidification liquid.

The liquid damper extends obliquely downward along the flow rectification portion and to a position below the intake airflow guide portion.

The flow rectification portion is provided inside with a flow guide rib, which is bent and extends to guide the airflow to flow toward the outlet of the flow rectification portion.

The tank body includes a bottom shell and an upper cover; the bottom shell and the upper cover enclose the internal chamber; the intake airflow guide portion is disposed on the upper cover; and when the humidification tank is rotated from the operating orientation by 180°, the first end or the second end is higher than the level of the humidification liquid.

The humidification tank further includes an outtake airflow guide portion having a third end and a fourth end; the third end is connected to the internal chamber; and the fourth end is hermetically connected to the airflow outlet.

The first end is provided with an air inlet; the fourth end is provided with a humidification outlet; the air inlet and the humidification outlet face the same side; the airflow inlet and the air inlet are arranged correspondingly; and the airflow outlet and the humidification outlet are arranged correspondingly.

When the technical solution according to the first aspect is adopted, the present invention has the following beneficial effects:
1. When the humidification tank according to the present invention is in use, the airflow sent by the blower enters the internal chamber through the intake airflow guide portion, and then it contacts the humidification liquid and gets moistened. The intake airflow guide portion includes a bent flow guide pipe having at least two bends, enabling a flowing path of airflow to be longer and more tortuous before the airflow contacts the humidification liquid. On one hand, this structure keeps down the howling sound generated during the flow process of the airflow, and reduces the impact force of the airflow on the inner wall of the bent flow guide pipe, thereby lowering the noise generated by this impact. On the other hand, the tortuous flowing path of airflow also lengthens the path of sound waves, making it difficult for the noise inside to be transmitted to the outside, thereby achieving good noise reduction and enhancing user convenience.
   Moreover, the humidification liquid in the tank body can only flow back in the direction opposite to the flowing direction of the airflow in the bent flow guide pipe that is bent and extends, which also lengthens the path for the liquid to flow back to the airflow inlet, thereby reducing the risk of back flow. Even if a part of the liquid flows back from the second end into the bent flow guide pipe, the liquid cannot flow directly to the first end due to the obstruction of the tortuous structure inside, which increases the difficulty of liquid flow. In addition, the internal chamber is a large-volume space for containing the humidification liquid, and it has only one chamber of a simple structure, with no complex partition structure, which not only saves production and processing costs, but also facilitates user use and greatly reduces the difficulty of operation. A user only needs to fill the tank body with the humidification liquid before use.
2. As a preferred implementation of the present invention, the bent flow guide pipe includes a first section, a second section, and a third section, which are connected in sequence; the first section and the third section extend along a first direction; and the second section extends in a direction opposite to the first direction. When the airflow flows from the first section to the second section, it needs to turn about 180° as the two sections extend in opposite directions. Similarly, when the airflow flows from the second section to the third section, it also needs to turn about 180°. The sound will be significantly reduced or even eliminated during such large-angle turns. Therefore, by designing the extension directions of the three sections properly, the airflow makes two large-angle turns in the bent flow guide pipe, achieving good noise reduction. Moreover, although the extension directions of the three sections are not exactly the same, the three sections are still parallel to each other, so that they can be disposed side by side, which helps to reduce the volume of the bent flow guide pipe and save space in the tank body, thereby helping to realize the miniaturization of the whole ventilator and making it more convenient for home use.
3. As a preferred implementation of the present invention, the third section is provided with an anti-backflow rib protruding upward on the bottom wall. The anti-backflow rib in the third section results in a height difference in the third section, so that when the humidification liquid flows back along the third section, it needs to flow over the anti-backflow rib. However, the liquid's own gravity and the resistance of the forward flowing airflow in the third section make it difficult for the humidification liquid to flow upward over the anti-backflow rib, so that the humidification liquid flowing back into the third section is hindered by the anti-backflow rib, and cannot further flow back into the second section, but is retained in the third section.
4. As a preferred implementation of the present invention, the intake airflow guide portion further includes a flow rectification portion arranged at the second end of the intake airflow guide portion; a liquid damper is provided on the lower side of the outlet of the flow rectification portion; and the liquid damper extends toward the first end and to a position below the intake airflow guide portion. The liquid damper can block the outlet below the flow rectification portion in the vertical direction, thereby reducing the risk of liquid splashing into the flow rectification portion and then entering the bent flow guide pipe. Moreover, the liquid damper can also guide the flowing direction of the airflow when it enters the internal chamber, so that the airflow can contact the humidification liquid in a direction parallel to or inclined to the level, to minimize the collision force between the airflow and the humidification liquid, thereby reducing noise. Otherwise, if the airflow collides with the humidification liquid in a direction perpendicular to the level, it will disturb the liquid and generate noise.
5. As a preferred implementation of the present invention, the humidification tank further includes an outtake airflow guide portion having a third end and a fourth end; and the intake airflow guide portion and the outtake airflow guide portion are configured as an integrated structure, and can be plug-in fitted as a whole with the airflow inlet mounting portion and the airflow outlet mounting portion respectively. The intake airflow guide portion and the outtake airflow guide portion are configured as an integrated structure, and can be plug-in fitted with the tank body, so that the two portions can be mounted in the tank body or removed from the tank body as a whole. In this way, the air duct assembly in the humidification tank can be mounted or removed as an independent module, which reduces the difficulty of processing and assembling for a higher assembling efficiency. In addition, this structure is also convenient for users to clean the intake airflow guide portion and the outtake airflow guide portion. Moreover, after the intake airflow guide portion and the outtake airflow guide portion are removed, the interior of the tank body is a complete cavity, thereby increasing the volume of the internal chamber, so that users can put in more humidification liquid, for a higher humidification efficiency.

When the technical solution according to the second aspect is adopted, the present invention has the following beneficial effects:
1. The humidification tank of the present invention is provided with an intake airflow guide portion, and the airflow sent by the blower first enters the intake airflow guide portion, passes through the air duct inside the intake airflow guide portion, and then enters the internal chamber, where it contacts the humidification liquid and gets moistened. According to the present invention, the flowing path of airflow in the intake airflow guide portion is so designed that it is bent and extends to form a first flow channel and a second flow channel, making a more tortuous backflow path for the humidification liquid in the flow guide pipe, so that when the humidification tank is rotated from the operating orientation to any direction by an angle of 90° or less, the humidification liquid cannot enter the first flow channel, and thus cannot flow to the first end, that is, the airflow outlet. Especially for a small portable household ventilator, even if the ventilator tips over as the user turns over in sleep or uses the ventilator improperly, the humidification liquid will not flow back, thus ensuring the safety and service life of the blower.
   The bending and extension of the flow guide pipe also enables the flowing path of airflow to be longer and more tortuous before the airflow contacts the humidification liquid. On one hand, this structure keeps down the howling sound generated during the flow process of the airflow, and reduces the impact force of the airflow on the inner wall of the flow guide pipe, thereby lowering the noise generated by this impact. On the other hand, the tortuous flowing path of airflow also lengthens the path of sound waves, making it difficult for the noise inside to be transmitted to the outside, thereby achieving good noise reduction and enhancing user convenience.
2. As a preferred implementation of the present invention, the intake airflow guide portion further includes a flow rectification portion disposed at the second end; the inlet of the flow rectification portion is connected to the outlet of the intake airflow guide portion; the outlet of the flow rectification portion is connected to the internal chamber of the humidification tank; and the position of the outlet of the flow rectification portion is lower than the position of the inlet of the flow rectification portion. The airflow flowing out of the outlet of the second flow channel enters the internal chamber and contacts the humidification liquid after rectification by the flow rectification portion. The position of the outlet of the flow rectification portion is lower, so that the outflow position of the airflow is closer to the level of the humidification liquid, thereby ensuring that more airflow can contact the humidification liquid for humidification, improving the humidification effect, and reducing the probability that the airflow flows directly from the humidification tank without humidification.
3. As a preferred implementation of the present invention, a liquid damper is provided on the lower side of the outlet of the flow rectification portion, and when the humidification tank is in the operating orientation, the lowest end of the liquid damper is higher than the level of the humidification liquid. The liquid damper can block the outlet below the flow rectification portion in the vertical direction, thereby reducing the risk of liquid splashing into the flow rectification portion and then entering the flow guide pipe. Moreover, the liquid damper can also guide the flowing direction of the airflow when it enters the internal chamber, so that the airflow can contact the humidification liquid in a direction parallel to or inclined to the level, to minimize the collision force between the airflow and the humidification liquid, thereby reducing noise. Otherwise, if the airflow collides with the humidification liquid in a direction perpendicular to the level, it will disturb the liquid and generate noise.
4. As a preferred implementation of the present invention, the tank body includes a bottom shell and an upper cover; the bottom shell and the upper cover enclose the internal chamber; the intake airflow guide portion is disposed on the upper cover; and when the humidification tank is rotated from the operating orientation by 180°, the first end or the second end is higher than the level of the humidification liquid. For the humidification liquid in the humidification tank, there is only one path to the airflow inlet. It can only flow through the flow guide pipe from the second end of the intake airflow guide portion to the first end, and finally to the airflow inlet. Therefore, when the humidification tank flips over by 180°, the humidification liquid accumulates in the space where the upper cover is located, and the first end or the second end is higher than the level of the humidification liquid, so that the humidification liquid needs to overcome its own gravity and flow upward before it comes to the first end. This structure greatly increases the difficulty of backflow, resulting in a better anti-backflow effect.
5. As a preferred implementation of the present invention, the humidification tank further includes an outtake airflow guide portion having a third end and a fourth end; the third end is connected to the internal chamber; and the fourth end is hermetically connected to the airflow outlet. The intake airflow guide portion and the outtake airflow guide portion can be plug-in fitted with the tank body, so that the two portions can be mounted in the tank body or removed from the tank body. In this way, the air duct assembly in the humidification tank can be mounted or removed as an independent module, which reduces the difficulty of processing and assembling for a higher assembling efficiency. In addition, this structure is also convenient for users to clean the intake airflow guide portion and the outtake airflow guide portion. Moreover, after the intake airflow guide portion and the outtake airflow guide portion are removed, the interior of the tank body is a complete cavity, thereby increasing the volume of the internal chamber, so that users can put in more humidification liquid, for a higher humidification efficiency.

### BRIEF DESCRIPTION OF DRAWINGS

The drawings illustrated herein are used to provide a further understanding of the present invention and constitute a part of the present invention. The exemplary embodiments of the present invention and descriptions thereof are not intended to limit the present invention, but only to explain the present invention. Among the drawings:
FIG. 1 is a schematic diagram of the internal structure of a humidification tank according to an implementation of the present invention;
FIG. 2 is a schematic diagram of the internal structure of the tank body in FIG. 1;
FIG. 3 is a schematic diagram of the external structure of the tank body in FIG. 1;
FIG. 4 is an exploded view of the structure of an intake airflow guide portion according to an implementation of the present invention;
FIG. 5 is a cross-sectional view of an intake airflow guide portion according to an implementation of the present invention;
FIG. 6 is a cross-sectional view of an intake airflow guide portion according to another implementation of the present invention;
FIG. 7 is a cross-sectional view of an intake airflow guide portion according to a further implementation of the present invention;
FIG. 8 is a cross-sectional view of a humidification tank in the operating orientation according to an implementation of the present invention;
FIG. 9 is a cross-sectional view of the humidification tank in FIG. 8 from another perspective;
FIG. 10 is a cross-sectional view of a humidification tank according to an implementation of the present invention after it rotates forward by 90°;
FIG. 11 is a schematic diagram of the structure of the intake airflow guide portion and the outtake airflow guide portion in FIG. 10;
FIG. 12 is a cross-sectional view of a humidification tank according to an implementation of the present invention after it rotates backward by 90°;
FIG. 13 is a schematic diagram of the structure of the intake airflow guide portion and the outtake airflow guide portion in FIG. 12;
FIG. 14 is a cross-sectional view of a humidification tank according to an implementation of the present invention after it rotates left by 90°;
FIG. 15 is a schematic diagram of the structure of the intake airflow guide portion and the outtake airflow guide portion in FIG. 14;
FIG. 16 is a cross-sectional view of a humidification tank according to an implementation of the present invention after it rotates right by 90°;
FIG. 17 is a schematic diagram of the structure of the intake airflow guide portion and the outtake airflow guide portion in FIG. 16;
FIG. 18 is an exploded view of the structure of an intake airflow guide portion according to an implementation of the present invention;
FIG. 19 is a cross-sectional view of an intake airflow guide portion according to an implementation of the present invention;
FIG. 20 is a cross-sectional view of an intake airflow guide portion according to another implementation of the present invention;
FIG. 21 is a cross-sectional view of an intake airflow guide portion according to a further implementation of the present invention;
FIG. 22 is a cross-sectional view of the humidification tank in FIG. 8 from another perspective;
FIG. 23 is a schematic diagram of the structure of the intake airflow guide portion and the outtake airflow guide portion in FIG. 10;
FIG. 24 is a schematic diagram of the structure of the intake airflow guide portion and the outtake airflow guide portion in FIG. 12;
FIG. 25 is a schematic diagram of the structure of the intake airflow guide portion and the outtake airflow guide portion in FIG. 14; and
FIG. 26 is a schematic diagram of the structure of the intake airflow guide portion and the outtake airflow guide portion in FIG. 16.

### Reference Numerals:

The reference numerals for the technical solution according to the first aspect described above are as follows:
1. tank body; 11. base; 12. cover; 121. rib; 13. internal chamber; 14. seal; 15. heat-conducting sheet; 16. airflow inlet; 17. airflow outlet;
2. intake airflow guide portion; 21. first end; 22 second end; 23. bent flow guide pipe; 231. first section; 232. second section; 233. third section; 2331. anti-backflow rib; 234. arc-shaped transition section; 235. partition; 236. lower shell; 237. upper shell; 24. notch; 25. flow rectification portion; 26. liquid damper; 27. flow guide rib;
3. outtake airflow guide portion; 31. third end; 32. fourth end;
4. humidification liquid.

The reference numerals for the technical solution according to the second aspect described above are as follows:
1. tank body; 11. bottom shell; 12. upper cover; 121. rib; 13. internal chamber; 14. seal; 15. heat-conducting sheet; 16. airflow inlet; 17. airflow outlet;
2. intake airflow guide portion; 21. first end; 22. second end; 23. flow guide pipe; 231. first flow channel; 2311. first section; 2312. second section; 232. second flow channel; 2321. anti-backflow rib; 233. arc-shaped transition section; 234. partition; 235. lower shell; 236. upper shell; 24. notch; 25. flow rectification portion; 26. liquid damper; 27. flow guide rib;
3. outtake airflow guide portion; 31. third end; 32. fourth end;
4. humidification liquid.

### DETAILED DESCRIPTION OF THE EMBODIMENTS

In order to more clearly explain the overall concept of the present invention, the technical solutions of the present invention are further detailed below with examples in conjunction with the drawings of the specification.

Many specific details are provided in the following description to facilitate a full understanding of the present invention. However, the present invention can also be implemented in other ways different from those described herein. Therefore, the scope of protection of the present invention is not limited by the specific embodiments disclosed below.

In addition, it should be understood in description of the present invention that the directions or position relationships such as "top", "bottom", "inside", and "outside" are based on those shown in the figures, and are used only for facilitating the description of the present invention and for simplified description, not for indicating or implying that the target devices or components must have a special direction and be structured and operated at the special direction, therefore they cannot be understood as the restrictions to the present invention.

In the present invention, unless otherwise specified or restricted, the terms "mount", "be connected to", "be connected with", and "fix" shall be understood as a general sense. For example, the connection may be fixed connection, removable connection, integrated connection, mechanical connection, electrical connection, communication, direct connection, indirect connection through intermediate media, connection between two components, or interaction between two components. For those of ordinary skill in the art, the specific meaning of the preceding terms in the present invention can be understood according to the specific situation.

In the present invention, unless otherwise specifically specified and defined, the first feature "above" or "below" the second feature may refer to direct contact between the first and second features, or indirect contact between the first and second features through intermediate media. In the description of the specification, descriptions with reference to the terms "implementation", "embodiment", "an embodiment", "example", or "specific example" mean that specific features, structures, materials or characteristics described in combination with the embodiment or example are contained in at least one embodiment or example of the present invention. In the specification, the schematic expressions of the above terms do not necessarily refer to the same embodiments or examples. Moreover, the specific features, structures, materials or characteristics described may be combined in any one or more embodiments or examples in a suitable manner.

According to a first aspect of the present invention, a humidification tank of a ventilator is provided, including but not limited to the following:

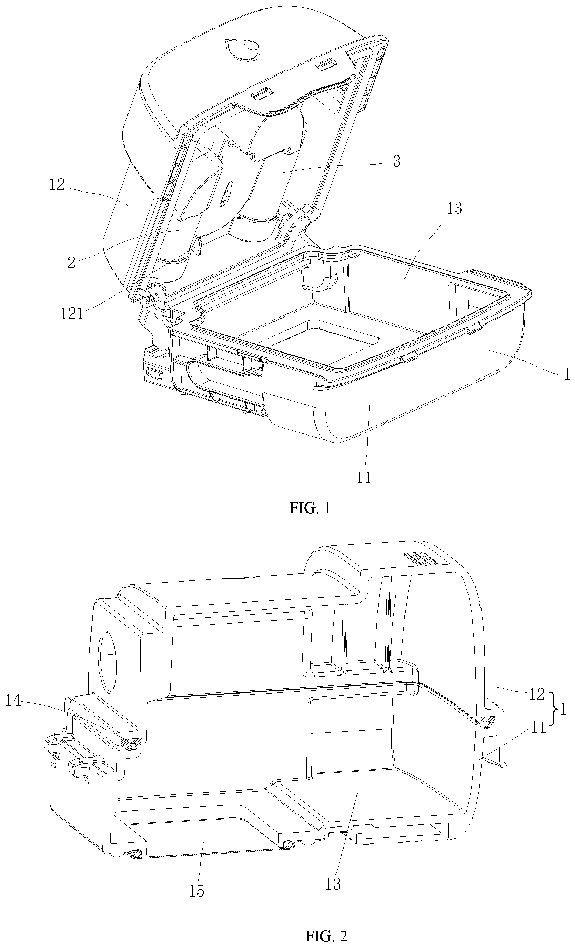

As shown in FIGS. 1 to 17, a humidification tank of a ventilator, including a tank body 1 and an internal chamber 13; the tank body 1 is provided with an airflow inlet 16 and an airflow outlet 17, and the internal chamber 13 is configured to be capable of containing a humidification liquid 4 and being communicated with an external air path by means of the airflow inlet 16 and the airflow outlet 17; the tank body 1 includes an insertion end and a graspable end; the insertion end can be inserted into the ventilator unit; the humidification tank further includes an intake airflow guide portion 2 having a first end 21 and a second end 22; the first end 21 is hermetically connected to the airflow inlet 16, and the second end 22 is connected to the internal chamber 13; and the intake airflow guide portion 2 includes a bent flow guide pipe 23 having at least two bends, such that the first end 21 and the second end 22 are located on two opposite sides.

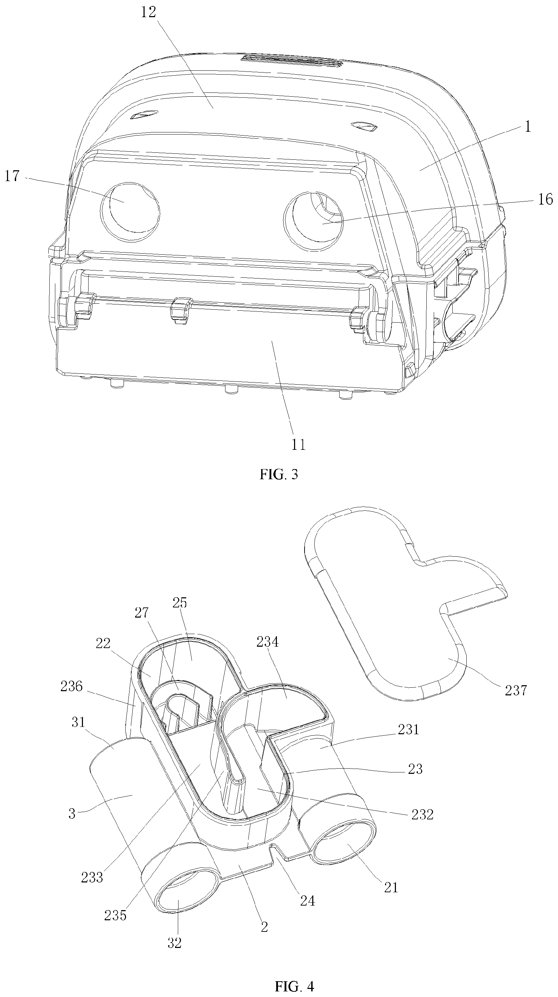

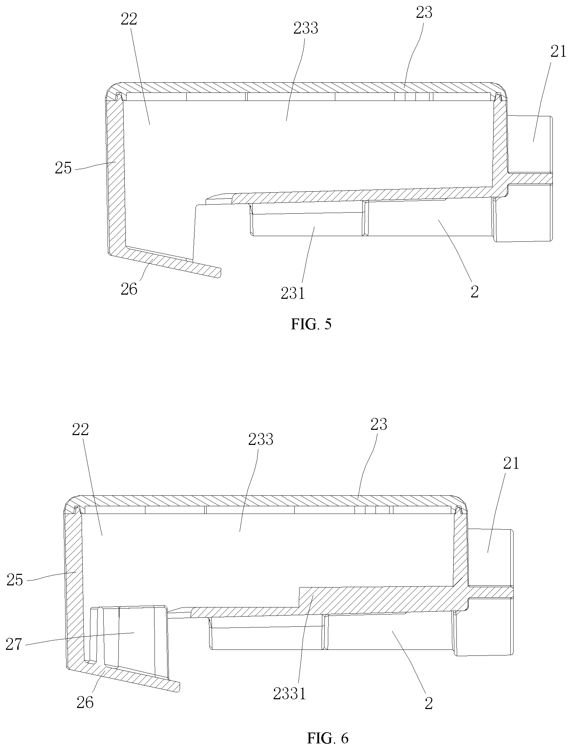

Further, as shown in FIG. 4 and FIG. 5, the first end 21 and the second end 22 are located on the left and right sides of the intake airflow guide portion 2 respectively, and also on the front and rear sides of the intake airflow guide portion 2 respectively.

It should be noted that the humidification liquid 4 is preferably water, which makes it easy for users to replace. Before use, municipal tap water or purified water can be taken into the tank body, so that the airflow contacts the water during use to complete the humidification. To save the use cost, users can easily replace or replenish the humidification liquid 4.

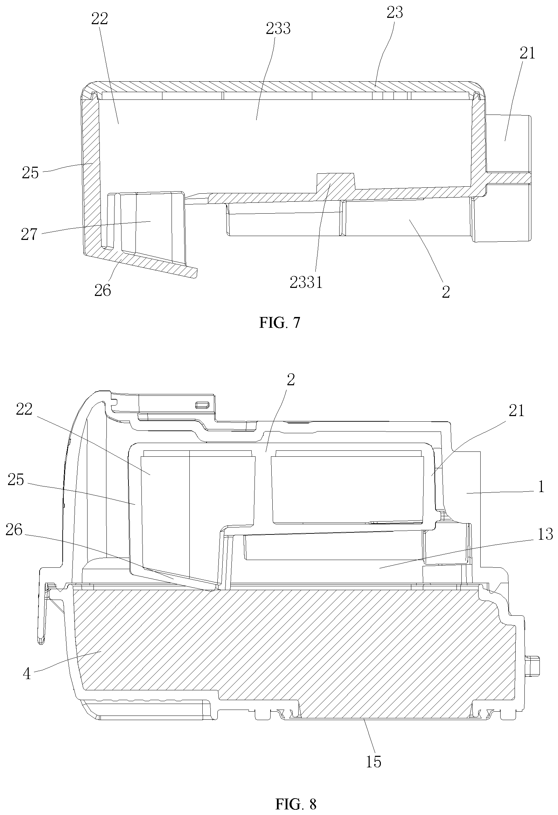

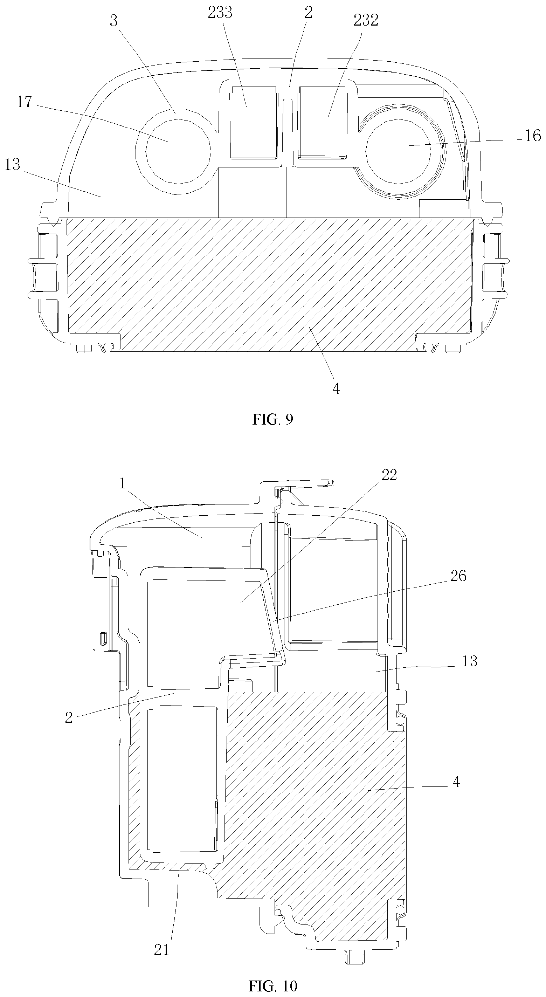

Optionally, as shown in FIG. 8 and FIG. 9, the intake airflow guide portion 2 is located above the humidification liquid 4, so that the intake airflow guide portion 2 and the humidification liquid 4 are disposed up and down. In this way, it is difficult for the humidification liquid 4 to flow back upward into the intake airflow guide portion 2 under its own gravity, thereby reducing the risk of back flow from the airflow inlet 16.

Optionally, the ventilator unit is provided with an opening on one side, and the insertion end of the tank body 1 can extend into the ventilator unit from the opening, while the graspable end is located outside the ventilator unit, so that it is convenient for users to directly mount the tank body 1 into the ventilator unit from one side of the ventilator unit, and to directly remove the tank body 1 from the ventilator unit by holding the graspable end located outside, with no need to completely disassemble the ventilator unit, thereby reducing the difficulty of assembling and disassembling, for a better user experience.

When the humidification tank according to the present invention is in use, the airflow sent by the blower enters the intake airflow guide portion 2 through the first end 21, flows through the bent flow guide pipe 23, enters the internal chamber 13 through the second end 22, and then it contacts the humidification liquid 4 and gets moistened. The intake airflow guide portion 2 includes a bent flow guide pipe 23 having at least two bends, enabling a flowing path of airflow to be longer and more tortuous before the airflow contacts the humidification liquid 4. On one hand, this structure keeps down the howling sound generated during the flow process of the airflow, and reduces the impact force of the airflow on the inner wall of the bent flow guide pipe 23, thereby lowering the noise generated by this impact. On the other hand, the tortuous flowing path of airflow also lengthens the path of sound waves, making it difficult for the noise inside to be transmitted to the outside, thereby achieving good noise reduction and enhancing user convenience.

In addition, the humidification liquid 4 in the tank body 1 can only flow back to the airflow inlet 16 in the direction opposite to the flowing direction of the airflow in the bent flow guide pipe 23 that is bent and extends, which also lengthens the path for the liquid to flow back to the airflow inlet 16, thereby reducing the risk of back flow. Even if a part of the liquid flows back from the second end 22 into the bent flow guide pipe 23, the liquid cannot flow directly to the first end 21 due to the obstruction of the tortuous structure inside, which increases the difficulty of liquid flow. In addition, the internal chamber 13 is a large-volume space for containing the humidification liquid, and it has only one chamber of a simple structure, with no complex partition structure, which not only saves production and processing costs, but also facilitates user use and greatly reduces the difficulty of operation. A user only needs to fill the tank body 1 with the humidification liquid 4 before use.

It should be noted that according to the present invention, there is no limit to the structure of the bent flow guide pipe 23. For example, the bent flow guide pipe 23 may extend in a Z-shape as a whole to form two bends respectively. Alternatively, the bent flow guide pipe may extend in an S-shape.

In an optional implementation, as shown in FIG. 4, the bent flow guide pipe 23 includes a first section 231, a second section 232, and a third section 233, which are connected in sequence; the first section 231 and the third section 233 extend along a first direction; and the second section 232 extends in a direction opposite to the first direction.

When the airflow flows from the first section 231 to the second section 232, it needs to turn about 180° as the two sections extend in opposite directions. Similarly, when the airflow flows from the second section 232 to the third section 233, it also needs to turn about 180°. The sound will be significantly reduced or even eliminated during such large-angle turns. Therefore, by designing the extension directions of the three sections properly, the airflow makes two large-angle turns in the bent flow guide pipe 23, achieving good noise reduction. Moreover, although the extension directions of the three sections are not exactly the same, the three sections are still parallel to each other, so that they can be disposed side by side, which helps to reduce the volume of the bent flow guide pipe 23 and save space in the tank body 1, thereby helping to realize the miniaturization of the whole ventilator and making it more convenient for home use.

Optionally, as shown in FIG. 4, arc-shaped transition sections 234 are provided between the first section 231 and the second section 232 and between the second section 232 and the third section 233, so that the airflow can be guided by the arc-shaped transition sections 234 to complete a soft and smooth transition at the two positions, to improve the flowing efficiency of the airflow, thereby preventing the occurrence of turbulent airflow, and reducing the noise generated by the violent collision between the airflow and the inner wall of the bent flow guide pipe 23.

Further, as shown in FIG. 4, the bent flow guide pipe 23 includes an upper shell 237 and a lower shell 236, and the upper shell 237 and the lower shell 236 enclose a channel for the airflow. The intake airflow guide portion 2 further includes an intake pipe fixed to one side of the bent flow guide pipe 23, and the intake pipe encloses at least a part of the first section 231. The upper shell 237 and the lower shell 236 can be fixedly connected by multiple means, such as gluing, welding, clamping, or fixing by screws. When they are detachably connected by means such as clamping or fixing by screws, a sealing ring should be provided at the connection between the two, to ensure the sealing of the airflow channel, thus preventing air leakage.

Further, as shown in FIG. 4, FIG. 5, and FIG. 11, the first section 231, the second section 232, and the third section 233 are disposed side by side, and the position of the third section 233 is higher than the position of the first section 231 and/or the position of the second section 232.

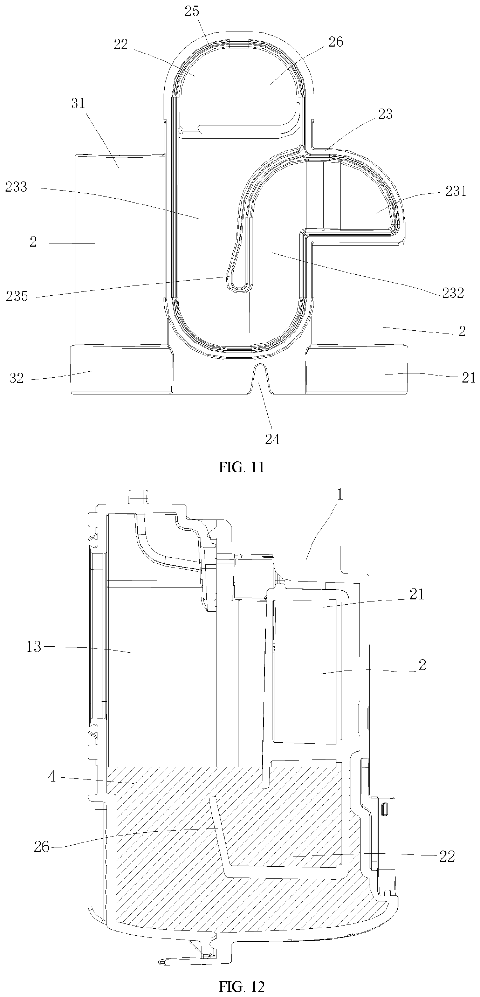

Furthermore, as shown in FIG. 11, the first section 231, the second section 232 and the third section 233 are disposed side by side in a direction perpendicular to the first direction, and a partition 235 is provided between every two adjacent sections to reduce the space occupied by the bent flow guide pipe 23, leaving a larger space in the internal chamber 13 for containing the humidification liquid 4, for a higher humidification efficiency.

It can be understood that the airflow inlet 16 is connected to the first section 231, and when the airflow flows in the bent flow guide pipe 23, it flows through the first section 231, the second section 232, and the third section 233 in sequence, and enters the internal chamber 13 from the second end 22. The third section 233 is located at a higher position, which is convenient for configuring any other flow guide structure at the second end 22, that is, the outlet of the third section 233, to improve the contact effect between the airflow and the humidification liquid 4. When the ventilator is in use, the intake airflow guide portion 2 is fixed above the internal chamber 13, and the humidification liquid 4 accumulates at the bottom of the internal chamber 13 under the action of gravity and below the intake airflow guide portion 2. Therefore, a higher position of the third section 233 can make a larger space between the two, while the level of the humidification liquid 4 remains unchanged, thereby facilitating the arrangement of the flow guide structure. In this way, there is still a gap between the flow guide structure or the second end 22 and the level of the humidification liquid 4, so that after the airflow enters the internal chamber 13, it contacts the surface of the humidification liquid 4 but does not go deeper below the level of the humidification liquid 4, to reduce the probability of being immersed in the humidification liquid 4, thereby avoiding the airflow from generating noise due to serious disturbance to the humidification liquid 4.

In an example, the heights of the first section 231, the second section 232, and the third section 233 increase sequentially. In another example, the first section 231 and the second section 232 are at the same height, and the third section 233 is higher than the first section and the second section. In a further example, the first section 231 is lower than the second section 232, and the third section 233 and the second section 232 are at the same height.

In addition, the third section 233 may extend horizontally, so that the overall height of the third section 233 is higher than the second section 232 and/or the first section 231. In an optional embodiment, as shown in FIG. 5 to FIG. 7, the bottom height of the third section 233 gradually decreases along the first direction.

The bottom height of the third section 233 gradually decreases from its inlet to its outlet. On one hand, this structure results in an enlarged outlet caliber of the third section 233, lowering the pressure and appropriately reducing the velocity of the airflow, thereby making a softer contact between the airflow and the humidification liquid 4 and reducing the noise generated. Moreover, the larger outlet caliber can increase the amount of air flowing out per unit time, so that more air flows into the internal chamber 13 and contacts the humidification liquid 4 for humidification, improving the humidification effect. On the other hand, as the bottom height of the third section 233 gradually increases along the backflow path of the humidification liquid 4, it is more difficult for the humidification liquid 4 to flow back. In other words, this structure increases the resistance for the humidification liquid 4 to flow back, which reduces the velocity of flow back, for a better anti-backflow effect.

In this embodiment, since the bottom of the third section 233 extends obliquely, the position of the third section 233 can be wholly higher than the first section 231 and/or the second section 232, that is to say, the lowest end of the bottom of the third section 233 is higher than the first section 231 and/or the second section 232. Another option is that a part of the third section 233 is higher than the first section 231 and/or the second section 232. For example, the lowest end of the third section 233 is at the same height as the first section 231 and/or the second section 232 or lower than the first section 231 and/or the second section 232, while the highest end of the third section 233 is higher than the first section 231 and/or the second section 232.

Optionally, as shown in FIG. 6 and FIG. 7, the third section 233 is provided with an anti-backflow rib 2331 protruding upward on the bottom wall of the third section 233.

The anti-backflow rib 2331 in the third section 233 results in a height difference in the third section 233, so that when the humidification liquid 4 flows back along the third section 233, it needs to flow over the anti-backflow rib 2331. However, the liquid's own gravity and the resistance of the forward flowing airflow in the third section 233 make it difficult for the humidification liquid 4 to flow upward over the anti-backflow rib 2331, so that the humidification liquid 4 flowing back into the third section 233 is hindered by the anti-backflow rib 2331, and cannot further flow back into the second section 232, but is retained in the third section 233.

As shown in FIG. 6, in an example, the bottom of the third section 233 is divided into two parts along the extension direction. The wall thickness of the part near the inlet of the third section 233 is greater, and the wall thickness of the part near the outlet of the third section 233 is smaller, so that a step is formed between the two parts, namely the anti-backflow rib 2331. In other examples, the bottom of the third section 233 can be divided into more parts, and the wall thicknesses of these parts gradually decrease along the extension direction of the third section 233, so that these parts of the third section 233 are disposed in a stepped manner to form more steps, for a better anti-backflow effect.

In another example, as shown in FIG. 7, a part of the bottom of the third section 233 protrudes upward to form an anti-backflow rib 2331, which does not extend to the inlet of the third section 233.

As an optional embodiment, the change in cross-sectional area between the first section 231 and the second section 232 and/or the change in cross-sectional area between the second section 232 and the third section 233 are/is less than 10%. In this embodiment, as the change in the cross-sectional area is limited for the three sections, the air duct in the bent flow guide pipe 23 is more uniform as a whole, to ensure a more uniform velocity of the airflow, thereby avoiding airflow turbulence and reducing noise.

Optionally, as shown in FIG. 4, the width of the third section 233 gradually increases along the first direction, to obtain a larger outlet of the third section 233, thereby increasing the volume of airflow entering the internal chamber 13 per unit time, and improving the air supply efficiency and humidification efficiency.

In an optional embodiment of this implementation, as shown in FIG. 4 to FIG. 7, the intake airflow guide portion 2 further includes a flow rectification portion 25 disposed at the second end 22; the inlet of the flow rectification portion 25 is connected to the outlet of the intake airflow guide portion 2; the outlet of the flow rectification portion 25 is connected to the internal chamber 13; and the position of the outlet of the flow rectification portion 25 is lower than the position of the inlet of the flow rectification portion 25.

The airflow flowing out of the outlet of the third section 233 enters the internal chamber 13 and contacts the humidification liquid 4 after rectification by the flow rectification portion 25. The position of the outlet of the flow rectification portion 25 is lower, so that the outflow position of the airflow is closer to the level of the humidification liquid 4, thereby ensuring that more airflow can contact the humidification liquid 4 for humidification, improving the humidification effect, and reducing the probability that the airflow flows directly from the humidification tank without humidification.

Further, as shown in FIG. 5 to FIG. 8, the position of the outlet of the flow rectification portion 25 is lower than the position of the first section 231, for a larger flow space after the airflow flows out of the flow rectification portion 25. The airflow can flow between the bottom of the bent flow guide pipe 23 and the level of the humidification liquid 4, to reduce the flow resistance to the airflow, thereby avoiding a drop in the flow efficiency that might be caused when the airflow is hindered by the outer wall of the first section 231 after flowing out of the outlet of the flow rectification portion 25.

As shown in FIG. 5, the bent flow guide pipe 23 extends downward at the end of the third section 233 to form the flow rectification portion 25, so that the airflow in the third section 233 flows downward under the guidance of the flow rectification portion 25, and enters the internal chamber 13 at a position closer to the level of the humidification liquid 4. When the humidification liquid 4 flows back, it needs to flow upward to pass the flow rectification portion 25 and enter the third section 233, which further increases the difficulty for the humidification liquid 4 to flow back, for a better anti-backflow effect.

Optionally, as shown in FIG. 5 to FIG. 8, the outlet of the flow rectification portion 25 is such configured that the airflow enters the internal chamber 13 in a direction different from the first direction. After the airflow in the third section 233 flows along the first direction to the flow rectification portion 25, it is rectified and guided by the flow rectification portion 25, and then enters the internal chamber 13 in a flow direction different from the first direction, so that the airflow turns again at the flow rectification portion 25, which further enables the flowing path of airflow to be longer and more tortuous, further reducing the generation of noise.

In an optional embodiment, as shown in FIG. 5 to FIG. 7, a liquid damper 26 is provided on the lower side of the outlet of the flow rectification portion 25, and the liquid damper 26 extends toward the first end 21 and to a position below the intake airflow guide portion 2.

The liquid damper 26 can block the outlet below the flow rectification portion 25 in the vertical direction, thereby reducing the risk of the humidification liquid 4 splashing into the flow rectification portion 25 and then entering the bent flow guide pipe 23. Moreover, the liquid damper 26 can also guide the flowing direction of the airflow when it enters the internal chamber 13, so that the airflow can contact the humidification liquid 4 in a direction parallel to or inclined to the level, to minimize the collision force between the airflow and the humidification liquid 4, thereby reducing noise. Otherwise, if the airflow collides with the humidification liquid 4 in a direction perpendicular to the level, it will disturb the liquid and generate noise.

Further, as shown in FIG. 5 to FIG. 8, the liquid damper 26 extends obliquely downward to form a flow guide slope on the surface of the liquid damper 26.

The flow guide slope makes the airflow contact the humidification liquid 4 below in an inclined downward direction, which not only reduces the noise generated by the violent collision between the airflow and the humidification liquid 4, but also enables the airflow after being rectified by the flow rectification portion 25 to be blown out more evenly when passing through the liquid damper 26, for a larger contact area with the humidification liquid 4, so that the airflow can fully contact the humidification liquid 4, thereby ensuring the humidification efficiency and improving the humidity of the airflow. In addition, when the humidification liquid 4 below flows back upward, due to the flow guide slope, the humidification liquid 4 needs to overcome its own gravity before it can flow upward, thereby reducing the risk of backflow. Under the guidance of the flow guide slope, the humidification liquid 4 flowing onto the liquid damper 26 will also flow back to the bottom under the action of gravity, so that it will not accumulate on the liquid damper 26.

The liquid damper 26 can also extend horizontally, so that the airflow enters the internal chamber 13 in a direction opposite to the first direction. The embodiments of the present invention are not limited to any particular implementation.

Optionally, as shown in FIG. 4, FIG. 6, and FIG. 7, the flow rectification portion 25 is provided with a flow guide rib 27, which is bent and extends to guide the airflow to flow toward the outlet of the flow rectification portion 25. The flow guide rib 27 is convexly bent toward the inlet of the flow rectification portion 25, to form a concave arc surface structure on one side facing the outlet of the flow rectification portion 25, which guides the airflow flowing thereto and the humidification liquid 4 flowing back thereto, so that the fluid flows downward into the internal chamber 13.

As shown in FIG. 6 and FIG. 7, a liquid damper 26 is provided below the flow rectification portion 25, and the flow guide rib 27 is disposed on the upper side of the liquid damper 26.

As an optional implementation of the present invention, as shown in FIG. 1, FIG. 4, and FIG. 11, the humidification tank further includes an outtake airflow guide portion 3 having a third end 31 and a fourth end 32; the third end 31 is connected to the internal chamber 13; and the fourth end 32 is hermetically connected to the airflow outlet 17.

After the airflow entering the internal chamber 13 contacts the humidification liquid 4 and gets moistened, it enters the outtake airflow guide portion 3 through the third end 31, flows out from the fourth end 32 through the airflow outlet 17, and then flows to the mask through the pipeline for use by the user. Further, as shown in FIG. 4 and FIG. 11, the outtake airflow guide portion 3 is an outlet pipe disposed on one side of the intake airflow guide portion 2. The first section 231, the second section 232, and the third section 233 of the bent flow guide pipe 23, as well as the outlet pipe, are disposed side by side in sequence.

Further, as shown in FIG. 1 and FIG. 3, the humidification tank includes a base 11 and a cover 12 that can be snap-fitted with each other, and the airflow inlet 16 and the airflow outlet 17 are disposed on the cover 12 and extend into the internal chamber 13 to form an airflow inlet mounting portion and an airflow outlet mounting portion respectively.

Optionally, as shown in FIG. 1, the cover 12 and the base 11 enclose the internal chamber 13, the space inside the cover 12 is configured to mount the intake airflow guide portion 2 and the outtake airflow guide portion 3, and the space inside the base 11 is for containing the humidification liquid 4. It can be understood that the amount of humidification liquid 4 in the internal chamber 13 is determined by the height of the base 11. When it is necessary to replenish the humidification liquid 4 into the internal chamber 13, the user needs to open the cover 12 to expose the inside of the tank body 1, and then add the humidification liquid 4 into the base 11. When the level is higher than the height of the base 11, the excess liquid will overflow the base 11. That is to say, when the humidification tank is in the normal operating orientation (when the humidification tank is stationary in the working state), the maximum level of the humidification liquid 4 in the internal chamber 13 is flush with the upper edge of the side wall of the base 11. Therefore, preferably, the height of the cover 12 is greater than the height of the base 11, so that the second end 22 of the intake airflow guide portion 2 is not lower than the upper edge of the side wall of the base 11. On one hand, this structure can provide sufficient mounting space for the intake airflow guide portion 2 and the outtake airflow guide portion 3 to prevent them from being immersed below the level after the cover is closed. On the other hand, when the humidification tank is in an inverted state, the humidification liquid 4 is in the space of the cover 11, where there are the intake airflow guide portion 2 and the outtake airflow guide portion 3, which also occupy a certain space, so that the level of the humidification liquid 4 will rise. Therefore, a higher height can compensate for a part of the space occupied by the intake airflow guide portion 2 and the outtake airflow guide portion 3, so that the level is lower than the second end 22 of the intake airflow guide portion 2, thereby preventing the humidification liquid 4 from flowing back from the airflow inlet 16 to the blower when the humidification tank is in an inverted state.

Optionally, as shown in FIG. 1, a seal 14 is provided between the base 11 and the cover 12 to ensure the sealing inside the tank body 135 after the cover is closed, to prevent the humidification liquid 4 and the airflow from leaking.

Further, as shown in FIG. 1, FIG. 4, and FIG. 11, the intake airflow guide portion 2 and the outtake airflow guide portion 3 are configured as an integrated structure, and can be plug-in fitted as a whole with the airflow inlet mounting portion and the airflow outlet mounting portion respectively.

The intake airflow guide portion 2 and the outtake airflow guide portion 3 are configured as an integrated structure, and can be plug-in fitted with the tank body 1, so that the two portions can be mounted in the tank body 1 or removed from the tank body 1 as a whole. In this way, the air duct assembly in the humidification tank can be mounted or removed as an independent module, which reduces the difficulty of processing and assembling for a higher assembling efficiency. In addition, this structure is also convenient for users to clean the intake airflow guide portion 2 and the outtake airflow guide portion 3. Moreover, after the intake airflow guide portion 2 and the outtake airflow guide portion 3 are removed, the interior of the tank body 1 is a complete cavity, thereby increasing the volume of the internal chamber 13, so that users can put in more humidification liquid 4, for a higher humidification efficiency.

Further, as shown in FIG. 1 and FIG. 3, the airflow inlet 16 and the airflow outlet 17 are located on the same side of the tank body 1, which further facilitates pipeline connecting and optimizes the pipeline layout.

Optionally, as shown in FIG. 1, FIG. 4, and FIG. 11, the intake airflow guide portion 2 or the outtake airflow guide portion 3 is provided with a positioning structure, and the cover 12 is provided with a matching structure matched with the positioning structure.

When the intake airflow guide portion 2 and the outtake airflow guide portion 3 are being mounted, the positioning structure and the matching structure can be taken as the reference for the convenience of mounting. In addition, this design can make the structure have a foolproof feature, so that users can mount the intake airflow guide portion 2 and the outtake airflow guide portion 3 to the cover 12 in correct postures, thereby reducing the difficulty of assembling and improving the convenience of use.

As shown in FIG. 1 and FIG. 11, the matching structure is a rib 121 provided on the cover 12, and the positioning structure is a notch 24 provided corresponding to the rib 121. They have the same shape or similar shapes, so that users can directly know the corresponding relationship between them. The positioning structure may be a rib, and correspondingly, the matching structure will be a notch. Alternatively, the positioning structure and the matching structure may be other structures respectively, as long as they can limit the positions of the intake airflow guide portion 2 and the outtake airflow guide portion 3 for mounting. Therefore, there is no limit to their structures herein.

Optionally, as shown in FIG. 2, the base 11 is further provided with a heat-conducting sheet 15, which is sealed and fixed to the base 11 to transfer the heat of the heating device to the internal chamber 13, so that the airflow in the internal chamber 13 is not only humidified but also warmed up, making the user's breathing more comfortable and improving the use experience.

The flowing path of the airflow of the ventilator when it passes the humidification tank according to the present invention is: The airflow sent by a blower enters the first end 21 of the intake airflow guide portion 2 through the airflow inlet 16; then it enters the bent flow guide pipe 23, and makes multiple turns inside the bent flow guide pipe 23, passing the first section 231, the second section 232, and the third section 233 in sequence; then it is rectified by the flow rectification portion 25 at the second end 22, and flows obliquely downward under the guidance of the liquid damper 26 to enter the internal chamber 13, where it contacts the humidification liquid 4 below and gets moistened; then the moistened airflow enters the outtake airflow guide portion 3 through the third end 31, and flows out from the fourth end 32 through the airflow outlet 17; and finally the airflow flows to the user end through the breathing tube and the mask, and is used by the user.

According to the present invention, the flowing path of airflow in the intake airflow guide portion 2 is so designed that when the humidification tank is rotated from an operating orientation to any direction by an angle of 90° or less, the humidification liquid 4 cannot flow to the first end 21, that is, the airflow outlet 16. Especially, for portable household small ventilators, even if such a ventilator tips over as the user turns over in sleep or uses the ventilator improperly, the humidification liquid 4 will not flow back, thus ensuring the safety and service life of the blower.

Further, taking the case when the extension direction of the first section 231, namely the first direction mentioned above, is the main viewing angle as an example, when the humidification tank is in the operating orientation, as shown in FIG. 9, the intake airflow guide portion 2 and the outtake airflow guide portion 3 are located above the humidification liquid 4, the airflow inlet 16 and the airflow outlet 17 face the front side, the first end 21 of the intake airflow guide portion 2 is located at the front side of the tank body 1, and the second end 22 is located at the rear side of the tank body 1. Then, the second end 22 of the intake airflow guide portion 2 is higher than the level of the humidification liquid 4, so that the humidification liquid 4 is at a lower position, thus reducing the risk of back flow.

When the humidification tank is rotated forward by 90°, as shown in FIG. 10 and FIG. 11, the humidification liquid 4 immerses the airflow inlet 16 and the airflow outlet 17. Since the airflow inlet 16 is hermetically connected to the first end 21, the humidification liquid 4 will naturally not flow out from the airflow inlet 16. In this state, the second end 22 is above the level of the humidification liquid 4, so that the humidification liquid 4 cannot enter the intake airflow guide portion 2 through the second end 22, and in other words, it cannot flow back. Even if a part of the humidification liquid 4 enters the bent flow guide pipe 23 from the second end 22 due to shaking, it will aggregate in the lower part. In the state as shown in FIG. 11, the bent flow guide pipe 23 has multiple sections extending upward, which will also hinder the liquid that has entered the bent flow guide pipe 23 from reaching the first end 21.

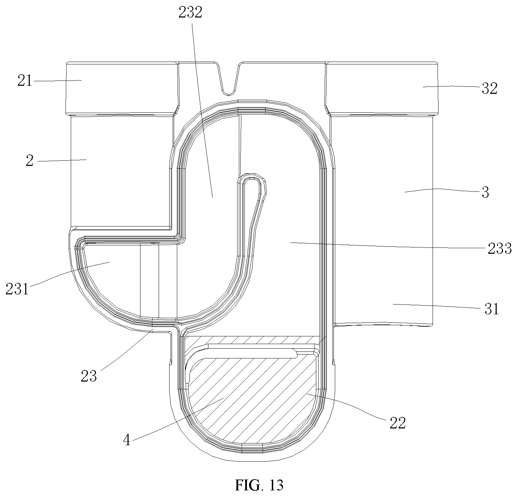

When the humidification tank is rotated backward by 90°, as shown in FIG. 12 and FIG. 13, the highest point of the partition 235 between the second section 232 and the third section 233 is higher than the level of the humidification liquid 4. This arrangement can ensure that when the humidification tank is in this direction, the humidification liquid 4 cannot enter the second section 232 or the first section 231. Even if the humidification tank reversely is rotated from this direction to the operating orientation, the humidification liquid 4 also cannot flow to the airflow inlet 16, further preventing the humidification liquid 4 from flowing back into the blower.

Optionally, as shown in FIG. 11, the first end 21 and the second end 22 of the intake airflow guide portion 2 are located on the left and right sides of the intake airflow guide portion 2 respectively, so that when the humidification tank is rotated leftward or rightward, the first end 21 and the second end 22 are at different heights.

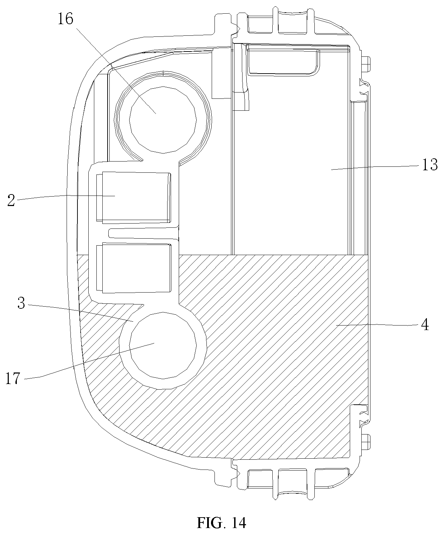

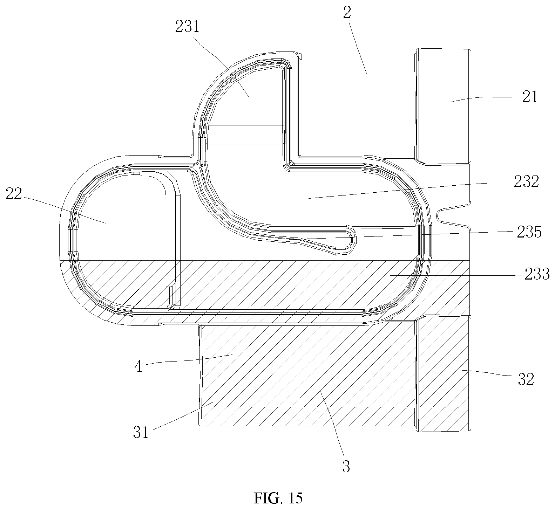

When the humidification tank is rotated left by 90°, as shown in FIG. 14 and FIG. 15, a part of the second end 22 is immersed below the level, and the first end 21 is higher than the level, so that the humidification liquid 4 also cannot flow upward to the first end 21. In addition, since there are partitions 235 between the first section 231 and the second section 232 and between the second section 232 and the third section 233, in this state, at least a part of the partition between the first section 231 and the second section 232 is above the level, and it can also hinder the humidification liquid 4 from flowing to the first end 21.

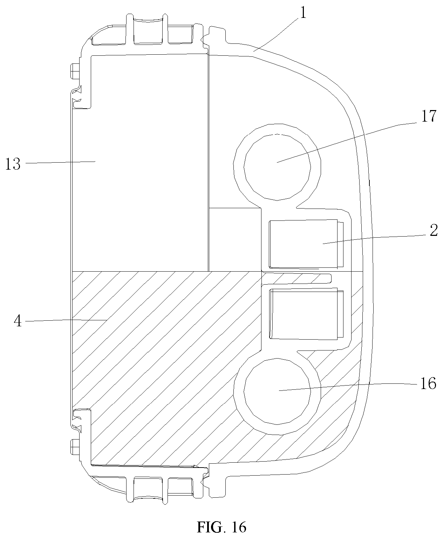

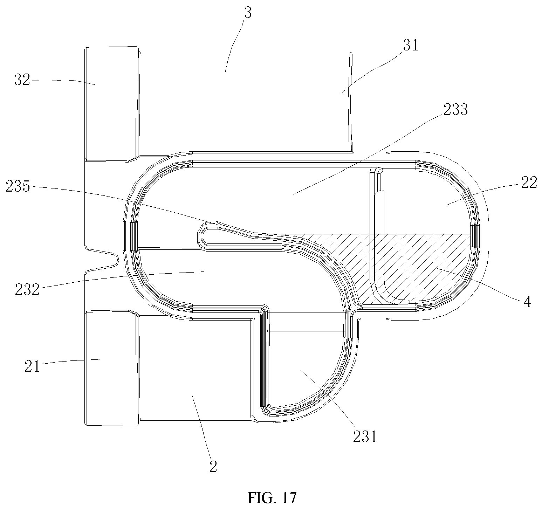

When the humidification tank is rotated right by 90°, as shown in FIG. 16 and FIG. 17, the first end 21 is immersed below the level, and a part of the second end 22 is higher than the level. Since the airflow inlet 16 is hermetically connected to the first end 21, the humidification liquid 4 will not flow out directly from the airflow inlet 16. At least a part of the partition between the third section 233 and the second section 232 is above the level, to hinder the humidification liquid 4 from entering the second end. Therefore, the humidification liquid 4 cannot flow in the bent flow guide pipe 23 to the first end 21.

In the above embodiment, the cases when the humidification tank is rotated front, back, left, and right by 90° are taken as examples to describe the anti-backflow effect of the humidification tank in four tip-over states. It can be understood that when the humidification tank is rotated by an angle of less than 90°, it can also have a good anti-backflow effect. In addition, the humidification tank can also maintain a good anti-backflow effect when it is rotated to any other direction, which is not listed here one by one.

In addition, it should be noted that in extreme cases, the posture of the humidification tank according to the present invention can be artificially and intentionally adjusted, making it possible for the humidification liquid inside to flow back from the airflow inlet 16, such as by collision or rapid and/or repeated rotation of the humidification tank. However, in the actual use of a ventilator, when the user finds that the ventilator body has tipped over, the user should adjust its posture in time to bring it back to the normal operating orientation. Therefore, the present invention is mainly intended to solve the backflow problem of humidification liquid when the humidification tank is in a common tip-over or flip-over state in actual use, but it cannot completely avoid the backflow of the humidification liquid caused by artificially swinging the humidification tank.

According to a second aspect of the present invention, a humidification tank of a ventilator is provided, including but not limited to the following:

As shown in FIG. 1 to FIG. 3, FIG. 8, FIG. 10, FIG. 12, FIG. 14, FIG. 16, and FIG. 18 to FIG. 26, a humidification tank of a ventilator, including a tank body 1 and an internal chamber 13; the tank body 1 is provided with an airflow inlet 16 and an airflow outlet 17, and the internal chamber 13 is configured to be capable of containing a humidification liquid 4 and being communicated with an external air path by means of the airflow inlet 16 and the airflow outlet 17; the tank body 1 includes an insertion end and a graspable end; the insertion end can be inserted into the ventilator unit; the humidification tank further includes an intake airflow guide portion 2 having a first end 21 and a second end 22; the first end 21 is hermetically connected to the airflow inlet 16, and the second end 22 is connected to the internal chamber 13; the intake airflow guide portion 2 includes a flow guide pipe 23 which is bent and extends; the flow guide pipe 23 includes a first flow channel 231 and a second flow channel 232, which are interconnected with each other; and the internal chamber 13 and the flow guide pipe 23 are such configured that when the humidification tank is rotated from the operating orientation to any direction by an angle of 90° or less, the humidification liquid 4 cannot enter the first flow channel 231, so that the humidification liquid 4 is prevented from flowing out of the airflow inlet 16.

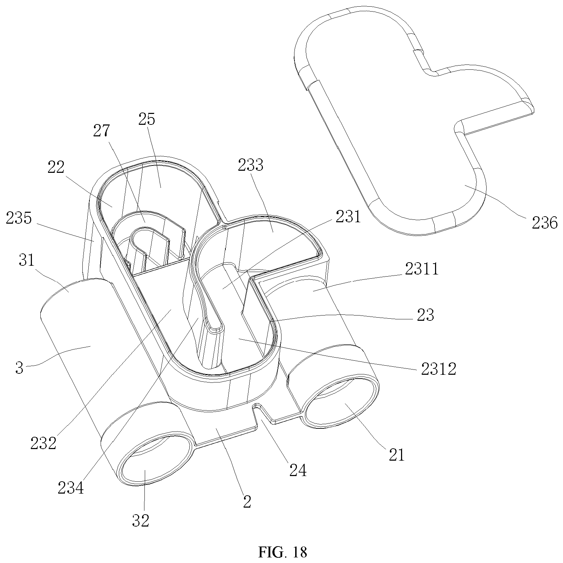

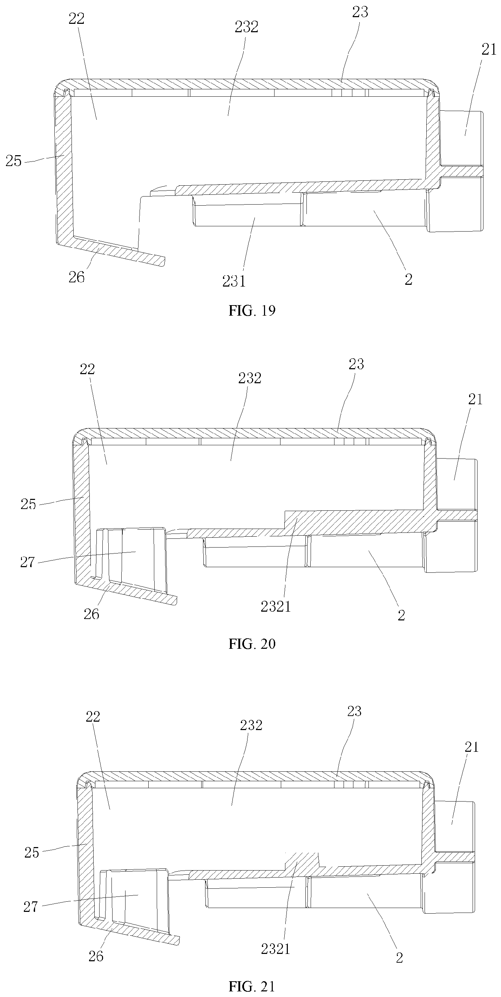

Further, as shown in FIG. 18 and FIG. 19, the first end 21 and the second end 22 are located on the left and right sides of the intake airflow guide portion 2 respectively, and also on the front and rear sides of the intake airflow guide portion 2 respectively, so that when the humidification tank is rotated forward, backward, left, or right by an angle of 90° or less, at least one of the first end 21 and the second end 22 is above the level of the humidification liquid 4.

It should be noted that the humidification liquid 4 is preferably water, which makes it easy for users to replace. Before use, municipal tap water or purified water can be taken into the tank body, so that the airflow contacts the water during use to complete the humidification. To save the use cost, users can easily replace or replenish the humidification liquid 4.

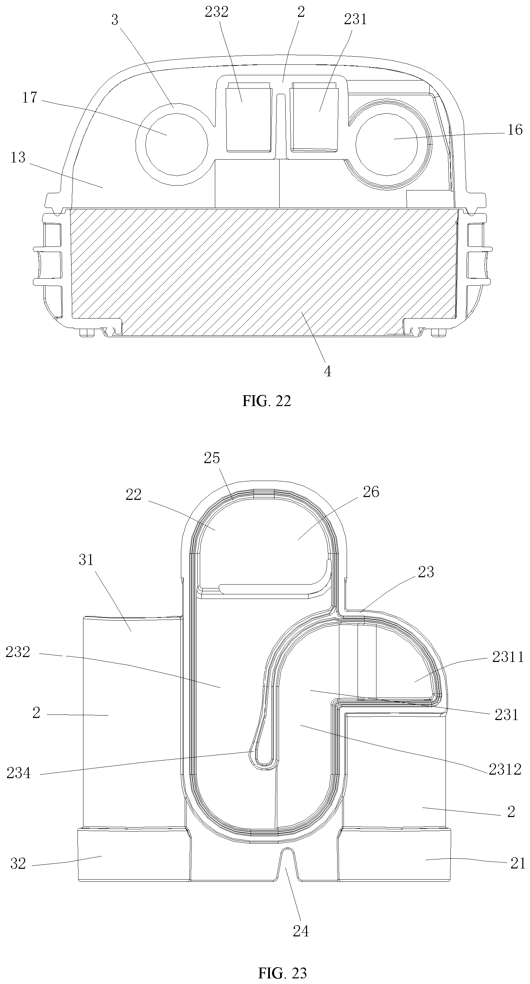

Optionally, as shown in FIG. 8 and FIG. 22, when the humidification tank is in the operating orientation, the intake airflow guide portion 2 is located above the humidification liquid 4, so that the intake airflow guide portion 2 and the humidification liquid 4 are disposed up and down. In this way, it is difficult for the humidification liquid 4 to flow back upward into the intake airflow guide portion 2 under its own gravity, thereby reducing the risk of back flow from the airflow inlet 16.

Optionally, the ventilator unit is provided with an opening on one side, and the insertion end of the tank body 1 can extend into the ventilator unit from the opening, while the graspable end is located outside the ventilator unit, so that it is convenient for users to directly mount the tank body 1 into the ventilator unit from one side of the ventilator unit, and to directly remove the tank body 1 from the ventilator unit by holding the graspable end located outside, with no need to completely disassemble the ventilator unit, thereby reducing the difficulty of assembling and disassembling, for a better user experience.

The humidification tank of the present invention is provided with an intake airflow guide portion 2, and the airflow sent by the blower first enters the intake airflow guide portion 2, passes through the air duct inside the intake airflow guide portion 2, and then enters the internal chamber 13, where it contacts the humidification liquid 4 and gets moistened. According to the present invention, the flowing path of the flow guide pipe 23 in the intake airflow guide portion 2 is so designed that it is bent and extends to form a first flow channel 231 and a second flow channel 232, making a more tortuous backflow path for the humidification liquid 4 in the flow guide pipe 23, so that when the humidification tank is rotated from the operating orientation to any direction by an angle of 90° or less, the humidification liquid 4 cannot enter the first flow channel 231, and thus cannot flow to the first end 21, that is, the airflow outlet 16. Especially, for portable household small ventilators, even if such a ventilator tips over as the user turns over in sleep or uses the ventilator improperly, the humidification liquid 4 will not flow back, thus ensuring the safety and service life of the blower.

The bending and extension of the flow guide pipe 23 also enables the flowing path of airflow to be longer and more tortuous before the airflow contacts the humidification liquid 4. On one hand, this structure keeps down the howling sound generated during the flow process of the airflow, and reduces the impact force of the airflow on the inner wall of the flow guide pipe 23, thereby lowering the noise generated by this impact. On the other hand, the tortuous flowing path of airflow also lengthens the path of sound waves, making it difficult for the noise inside to be transmitted to the outside, thereby achieving good noise reduction and enhancing user convenience.

Further, as shown in FIG. 22, take the case when the extension direction of the second flow channel 231 is the main viewing angle as an example. When the humidification tank is in the operating orientation, as shown in FIG. 22, the intake airflow guide portion 2 is located above the humidification liquid 4, the airflow inlet 16 and the airflow outlet 17 face the front side, the first end 21 of the intake airflow guide portion 2 is located at the front side of the tank body 1, and the second end 22 is located at the rear side of the tank body 1. Then, the second end 22 of the intake airflow guide portion 2 is higher than the level of the humidification liquid 4, so that the humidification liquid 4 is at a lower position, thus reducing the risk of back flow.

The following paragraphs take the cases when the humidification tank is rotated forward, backward, left, and right by 90° as examples to describe the anti-backflow principle and effect of the humidification tank after rotation:

In some implementations, the airflow inlet 16 is disposed on the front side of the tank body 1, both the first flow channel 231 and the second flow channel 232 extend along the front-to-back direction of the tank body 1, and when the humidification tank is rotated forward from the operating orientation by an angle of 90° or less, the second end 22 is higher than the level of the humidification liquid 4.

Further, when the humidification tank is rotated forward by 90°, as shown in FIG. 10 and FIG. 23, the humidification liquid 4 immerses the airflow inlet 16 and the airflow outlet 17. Since the airflow inlet 16 is hermetically connected to the first end 21, the humidification liquid 4 will naturally not flow out from the airflow inlet 16. In this state, the second end 22 is above the level of the humidification liquid 4, so that the humidification liquid 4 cannot enter the intake airflow guide portion 2 through the second end 22, and in other words, it cannot flow back. Even if a part of the humidification liquid 4 enters the flow guide pipe 23 from the second end 22 due to shaking, it will aggregate in the lower part. In the state as shown in FIG. 23, the flow guide pipe 23 has multiple sections extending upward, which will also hinder the liquid that has entered the flow guide pipe 23 from reaching the first end 21.

In some implementations, when the humidification tank is rotated backward from the working orientation by an angle of 90° or less, the first end 21 is higher than the level of the humidification liquid 4.

Further, the flow guide pipe 23 is provided inside with a partition 234, which is located between the first flow channel 231 and the second flow channel 232. When the humidification tank is rotated backward by 90°, the highest point of the partition 234 is higher than the level of the humidification liquid 4. This arrangement can ensure that when the humidification tank is in this direction, the humidification liquid 4 cannot enter the first flow channel 231. Even if the humidification tank reversely is rotated from this direction to the operating orientation, the humidification liquid 4 also cannot flow to the airflow inlet 16, further preventing the humidification liquid 4 from flowing back into the blower.

In some implementations, the airflow inlet 16 is disposed on the front side of the tank body 1, the flow guide pipe 23 is provided inside with a partition 234, which is located between the first flow channel 231 and the second flow channel 232. When the humidification tank is rotated leftward or rightward from the operating orientation by an angle of 90° or less, at least a part of the partition 234 is higher than the level of the humidification liquid 4.

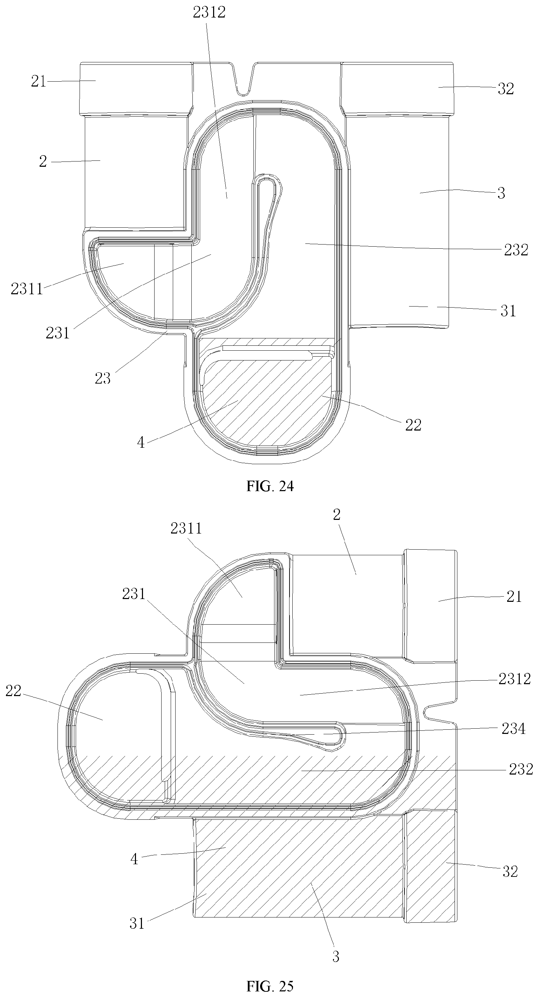

Further, when the humidification tank is rotated left by 90°, as shown in FIG. 14 and FIG. 25, a part of the second end 22 is immersed below the level, and the first end 21 is higher than the level, so that the humidification liquid 4 also cannot flow upward to the first end 21. In this state, at least a part of the partition 234 between the first flow channel 231 and the second flow channel 232 is above the level, and it can also hinder the humidification liquid 4 from flowing to the first end 21.

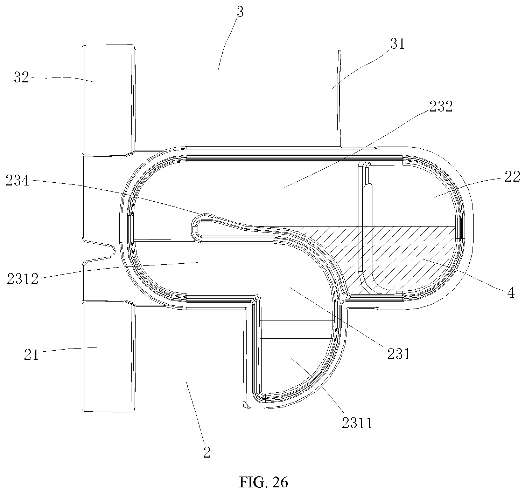

When the humidification tank is rotated right by 90°, as shown in FIG. 16 and FIG. 26, the first end 21 is immersed below the level, and a part of the second end 22 is higher than the level. Since the airflow inlet 16 is hermetically connected to the first end 21, the humidification liquid 4 will not flow out directly from the airflow inlet 16. At least a part of the partition between the first flow channel 231 and the second flow channel 232 is above the level, to hinder the humidification liquid 4 from entering the second end 22. Therefore, the humidification liquid 4 cannot flow in the flow guide pipe 23 to the first end 21. In the above implementation, the cases when the humidification tank is rotated front, back, left, and right by 90° are taken as examples to describe the anti-backflow effect of the humidification tank in four tip-over states. It can be understood that when the humidification tank is rotated by an angle of less than 90°, it can also have a good anti-backflow effect. In addition, the humidification tank can also maintain a good anti-backflow effect when it is rotated to any other direction, which is not listed here one by one.

In addition, it should be noted that in extreme cases, the posture of the humidification tank according to the present invention can be artificially and intentionally adjusted, making it possible for the humidification liquid inside to flow back from the airflow inlet 16, such as by collision or rapid and/or repeated rotation of the humidification tank. However, in the actual use of a ventilator, when the user finds that the ventilator body has tipped over, the user should adjust its posture in time to bring it back to the normal operating orientation. Therefore, the present invention is mainly intended to solve the backflow problem of humidification liquid when the humidification tank is in a common tip-over or flip-over state in actual use, but it cannot completely avoid the backflow of the humidification liquid caused by artificially swinging the humidification tank.

As an optional implementation of the present invention, as shown in FIG. 18 and FIG. 23, the first flow channel 231 includes a first section 2311 and a second section 2312 disposed sequentially and in parallel, and the second section 2312 is butt-jointed and communicated with the second flow channel 232, so that the flow guide pipe 23 can extend in a Z-shape as a whole to form two bends respectively. Alternatively, the flow guide pipe 23 extends in an S-shape.

In an optional implementation, as shown in FIG. 18, the first section 2311, the second section 2312, and the second flow channel 232 are connected in sequence; the first section 2311 and the second flow channel 232 extend along the first direction; and the second section 2312 extends in a direction opposite to the first direction.

When the airflow flows from the first section 2311 to the second section 2312, it needs to turn about 180° as the two sections extend in opposite directions. Similarly, when the airflow flows from the second section 2312 to the second flow channel 232, it also needs to turn about 180°. The sound will be significantly reduced or even eliminated during such large-angle turns. Therefore, by designing the extension directions of the three sections properly, the airflow makes two large-angle turns in the flow guide pipe 23, achieving good noise reduction. Moreover, although the extension directions of the three sections are not exactly the same, the three sections are still parallel to each other, so that they can be disposed side by side, which helps to reduce the volume of the flow guide pipe 23 and save space in the tank body 1, thereby helping to realize the miniaturization of the whole ventilator and making it more convenient for home use.

Optionally, as shown in FIG. 18, arc-shaped transition sections 233 are provided between the first section 2311 and the second section 2312, and between the second section 2312 and the second flow channel 232, so that the airflow can be guided by the arc-shaped transition sections 233 to complete a soft and smooth transition at the two positions, to improve the flowing efficiency of the airflow, thereby preventing the occurrence of a turbulent airflow, and reducing the noise generated by the violent collision between the airflow and the inner wall of the flow guide pipe 23.

Further, as shown in FIG. 18, the flow guide pipe 23 includes an upper shell 236 and a lower shell 235, and the upper shell 236 and the lower shell 235 enclose a channel for the airflow. The intake airflow guide portion 2 further includes an intake pipe fixed to one side of the flow guide pipe 23, and the intake pipe encloses at least a part of the first section 2311. The upper shell 236 and the lower shell 235 can be fixedly connected by multiple means, such as gluing, welding, clamping, or fixing by screws. When they are detachably connected by means such as clamping or fixing by screws, a sealing ring should be provided at the connection between the two, to ensure the sealing of the airflow channel, thus preventing air leakage.

Further, as shown in FIG. 18, FIG. 19, and FIG. 23, the first flow channel 231 and the second flow channel 232 are disposed side by side, and the position of a part of the second flow channel 232 is higher than that of the first flow channel 231.

Further, as shown in FIG. 23, the first flow channel 231 and the second flow channel 232 are disposed side by side in a direction perpendicular to the first direction, and a partition 234 is provided between the two flow channels to reduce the space occupied by the flow guide pipe 23, leaving a larger space in the internal chamber 13 for containing the humidification liquid 4, for a higher humidification efficiency.

Therefore, a higher position of the second flow channel 232 can make a larger space between the two, while the level of the humidification liquid 4 remains unchanged, thereby facilitating the arrangement of the flow guide structure. In this way, there is still a gap between the flow guide structure or the second end 22 and the level of the humidification liquid 4, so that after the airflow enters the internal chamber 13, it contacts the surface of the humidification liquid 4 but does not go deeper below the level of the humidification liquid 4, to reduce the probability of being immersed in the humidification liquid 4, thereby avoiding the airflow from generating noise due to serious disturbance to the humidification liquid 4.

Optionally, as shown in FIG. 20 and FIG. 21, the second flow channel 232 is provided with an anti-backflow rib 2321 protruding upward on the bottom wall.

The anti-backflow rib 2321 in the second flow channel 232 results in a height difference in the second flow channel 232, so that when the humidification liquid 4 flows back along the second flow channel 232, it needs to flow over the anti-backflow rib 2321. However, the liquid's own gravity and the resistance of the forward flowing airflow in the second flow channel 232 make it difficult for the humidification liquid 4 to flow upward over the anti-backflow rib 2321, so that the humidification liquid 4 flowing back into the second flow channel 232 is hindered by the anti-backflow rib 2321, and cannot further flow back into the first flow channel 231, but is retained in the second flow channel 232.

Further, as shown in FIG. 20, in one example, the bottom of the second flow channel 232 is divided into two parts along the extension direction. The wall thickness of the part near the inlet of the second flow channel 232 is greater, and the wall thickness of the part near the outlet of the second flow channel 232 is smaller, so that a step is formed between the two parts to form an anti-backflow rib 2321. In other examples, the bottom of the second flow channel 232 can be divided into more parts, and the wall thicknesses of these parts gradually decrease along the extension direction of the second flow channel 232, so that these parts of the second flow channel 232 are disposed in a stepped manner to form more steps, for a better anti-backflow effect.

In another example, as shown in FIG. 21, a part of the bottom of the second flow channel 232 protrudes upward to form an anti-backflow rib 2321, which does not extend to the inlet of the second flow channel 232.

As an embodiment, the change in cross-sectional area between the first flow channel 231 and the second flow channel 232 is less than 10%. In this embodiment, as the change in the cross-sectional area is limited for the two flow channels, the air duct in the flow guide pipe 23 is more uniform as a whole, to ensure a more uniform velocity of the airflow, thereby avoiding airflow turbulence and reducing noise.

Optionally, as shown in FIG. 18, the width of the second flow channel 232 gradually increases along the first direction, to obtain a larger outlet of the second flow channel 232, thereby increasing the volume of airflow entering the internal chamber 13 per unit time, and improving the air supply efficiency and humidification efficiency.

As an embodiment of the present invention, as shown in FIG. 18 to FIG. 21, the intake airflow guide portion 2 further includes a flow rectification portion 25 disposed at the second end 22; the inlet of the flow rectification portion 25 is connected to the outlet of the intake airflow guide portion 2; the outlet of the flow rectification portion 25 is connected to the internal chamber 13; and the position of the outlet of the flow rectification portion 25 is lower than the position of the inlet of the flow rectification portion 25.

The airflow flowing out of the outlet of the second flow channel 232 enters the internal chamber 13 and contacts the humidification liquid 4 after rectification by the flow rectification portion 25. The position of the outlet of the flow rectification portion 25 is lower, so that the outflow position of the airflow is closer to the level of the humidification liquid 4, thereby ensuring that more airflow can contact the humidification liquid 4 for humidification, improving the humidification effect, and reducing the probability that the airflow flows directly from the humidification tank without humidification.

Further, as shown in FIG. 19 to FIG. 21 and FIG. 8, the position of the outlet of the flow rectification portion 25 is lower than the position of the first flow channel 231, for a larger flow space after the airflow flows out of the flow rectification portion 25. The airflow can flow between the bottom of the flow guide pipe 23 and the level of the humidification liquid 4, to reduce the flow resistance to the airflow, thereby avoiding a drop of the flow efficiency that might be caused when the airflow is hindered by the outer wall of the first flow channel 231 after flowing out of the outlet of the flow rectification portion 25.

Further, as shown in FIG. 19, the flow guide pipe 23 extends downward at the end of the second flow channel 232 to form the flow rectification portion 25, so that the airflow in the second flow channel 232 flows downward under the guidance of the flow rectification portion 25, and enters the internal chamber 13 at a position closer to the level of the humidification liquid 4. When the humidification liquid 4 flows back, it needs to flow upward to pass the flow rectification portion 25 and enter the second flow channel 232, which further increases the difficulty for the humidification liquid 4 to flow back, for a better anti-backflow effect.

Optionally, as shown in FIG. 19 to FIG. 21 and FIG. 8, the outlet of the flow rectification portion 25 is such configured that the airflow enters the internal chamber 13 in a direction different from the first direction. After the airflow in the second flow channel 232 flows along the first direction to the flow rectification portion 25, it is rectified and guided by the flow rectification portion 25, and then enters the internal chamber 13 in a flow direction different from the first direction, so that the airflow turns again at the flow rectification portion 25, which further enables the flowing path of airflow to be longer and more tortuous, further reducing the generation of noise.

In an optional embodiment, as shown in FIG. 19 to FIG. 21, a liquid damper 26 is provided on the lower side of the outlet of the flow rectification portion 25, and the liquid damper 26 extends toward the first end 21 and to a position below the intake airflow guide portion 2.

The liquid damper 26 can block the outlet below the flow rectification portion 25 in the vertical direction, thereby reducing the risk of the humidification liquid 4 splashing into the flow rectification portion 25 and then entering the flow guide pipe 23. Moreover, the liquid damper 26 can also guide the flowing direction of the airflow when it enters the internal chamber 13, so that the airflow can contact the humidification liquid 4 in a direction parallel to or inclined to the level, to minimize the collision force between the airflow and the humidification liquid 4, thereby reducing noise. Otherwise, if the airflow collides with the humidification liquid 4 in a direction perpendicular to the level, it will disturb the liquid and generate noise.

Further, as shown in FIG. 19 to FIG. 21 and FIG. 8, the liquid damper 26 extends obliquely downward to form a flow guide slope on the surface of the liquid damper 26.

The flow guide slope makes the airflow contact the humidification liquid 4 below in an inclined downward direction, which not only reduces the noise generated by the violent collision between the airflow and the humidification liquid 4, but also enables the airflow after being rectified by the flow rectification portion 25 to be blown out more evenly when passing through the liquid damper 26, for a larger contact area with the humidification liquid 4, so that the airflow can fully contact the humidification liquid 4, thereby ensuring the humidification efficiency and improving the humidity of the airflow. In addition, when the humidification liquid 4 below flows back upward, due to the flow guide slope, the humidification liquid 4 needs to overcome its own gravity before it can flow upward, thereby reducing the risk of backflow. Under the guidance of the flow guide slope, the humidification liquid 4 flowing onto the liquid damper 26 will also flow back to the bottom under the action of gravity, so that it will not accumulate on the liquid damper 26.

The liquid damper 26 can also extend horizontally, so that the airflow enters the internal chamber 13 in a direction opposite to the first direction. The embodiments of the present invention are not limited to any particular implementation.

Optionally, as shown in FIG. 18, FIG. 20, and FIG. 21, the flow rectification portion 25 is provided with a flow guide rib 27, which is bent and extends to guide the fluid to flow toward the outlet of the flow rectification portion 25. The flow guide rib 27 is convexly bent toward the inlet of the flow rectification portion 25, to form a concave arc surface structure on one side facing the outlet of the flow rectification portion 25, which guides the airflow flowing thereto and the humidification liquid 4 flowing back thereto, so that the fluid flows downward into the internal chamber 13.

Further, as shown in FIG. 20 and FIG. 21, a liquid damper 26 is provided below the flow rectification portion 25, and the flow guide rib 27 is disposed on the upper side of the liquid damper 26.

In some implementations, the tank body 1 includes a bottom shell 11 and an upper cover 12; the bottom shell 11 and the upper cover 12 enclose the internal chamber 13; the intake airflow guide portion 2 is disposed on the upper cover 12; and when the humidification tank is rotated from the operating orientation by 180°, the first end 21 or the second end 22 is higher than the level of the humidification liquid 4.

For the humidification liquid 4 in the humidification tank, there is only one path to the airflow inlet 16. It can only flow through the flow guide pipe 23 from the second end 22 of the intake airflow guide portion 2 to the first end 21, and finally to the airflow inlet 16. Therefore, when the humidification tank is rotated180°, the humidification liquid 4 accumulates in the space where the upper shell 12 is located, and the first end 21 or the second end 22 is higher than the level of the humidification liquid 4, so that the humidification liquid 4 needs to overcome its own gravity and flow upward before it can flow to the first end 21. This structure greatly increases the difficulty of backflow, resulting in a better anti-backflow effect.

For example, when the first end 21 is above the level of the humidification liquid 4, although the humidification liquid 4 can enter the flow guide pipe 23 through the second end 22, it cannot flow upward to the first end 21. When the second end 22 is above the level of the humidification liquid 4, the level of the humidification liquid 4 is lower than the opening of the second end 22, so that it cannot enter the flow guide pipe 23.

As an implementation of the present invention, as shown in FIG. 1, FIG. 18, and FIG. 23, the humidification tank further includes an outtake airflow guide portion 3 having a third end 31 and a fourth end 32; the third end 31 is connected to the internal chamber 13; and the fourth end 32 is hermetically connected to the airflow outlet 17.

After the airflow entering the internal chamber 13 contacts the humidification liquid 4 and gets moistened, it enters the outtake airflow guide portion 3 through the third end 31, flows out from the fourth end 32 through the airflow outlet 17, and then flows to the mask through the pipeline for use by the user. Specifically, as shown in FIG. 4 and FIG. 11, the outtake airflow guide portion 3 is an outlet pipe disposed on one side of the intake airflow guide portion 2. The first flow channel 231 and the second flow channel 232 of the flow guide pipe 23, as well as the outlet pipe, are disposed side by side in sequence.

Further, as shown in FIG. 1 and FIG. 3, the airflow inlet 16 and the airflow outlet 17 are disposed on the upper cover 12 and extend to the internal chamber 13 to form an airflow inlet mounting portion and an airflow outlet mounting portion respectively.

Optionally, as shown in FIG. 1, the upper cover 12 and the bottom shell 11 enclose the internal chamber 13, the space inside the upper cover 12 is configured to mount the intake airflow guide portion 2 and the outtake airflow guide portion 3, and the space inside the bottom shell 11 is for containing the humidification liquid 4. It can be understood that the amount of humidification liquid 4 in the internal chamber 13 is determined by the height of the bottom shell 11. When it is necessary to replenish the humidification liquid 4 into the internal chamber 13, the user needs to open the upper cover 12 to expose the inside of the tank body 1, and then add the humidification liquid 4 into the bottom shell 11. When the level is higher than the height of the bottom shell 11, the excess liquid will overflow the bottom shell 11. That is to say, when the humidification tank is in the normal operating orientation (when the humidification tank is stationary in the working state), the maximum level of the humidification liquid 4 in the internal chamber 13 is flush with the upper edge of the side wall of the bottom shell 11. Therefore, optionally, the height of the upper cover 12 is greater than the height of the bottom shell 11, so that the second end 22 of the intake airflow guide portion 2 is not lower than the upper edge of the side wall of the bottom shell 11. On one hand, this structure can provide sufficient mounting space for the intake airflow guide portion 2 and the outtake airflow guide portion 3 to prevent them from being immersed below the level after the cover is closed. On the other hand, when the humidification tank is in an inverted state, the humidification liquid 4 is in the space of the upper cover 12, where there are the intake airflow guide portion 2 and the outtake airflow guide portion 3, which also occupy a certain space, so that the level of the humidification liquid 4 will rise. Therefore, a higher height can compensate for a part of the space occupied by the intake airflow guide portion 2 and the outtake airflow guide portion 3, so that the level is lower than the second end 22 of the intake airflow guide portion 2, thereby preventing the humidification liquid 4 from flowing back from the airflow inlet 16 to the blower when the humidification tank is in an inverted state.

Optionally, as shown in FIG. 2, a seal 14 is provided between the bottom shell 11 and the upper cover 12 to ensure the sealing inside the tank body 1 after the cover is closed, to prevent the leakage of the humidification liquid 4 and the airflow.

Further, as shown in FIG. 1, FIG. 18, and FIG. 23, the intake airflow guide portion 2 and the outtake airflow guide portion 3 are configured as an integrated structure, and can be plug-in fitted as a whole with the airflow inlet mounting portion and the airflow outlet mounting portion respectively.

The intake airflow guide portion 2 and the outtake airflow guide portion 3 are configured as an integrated structure, and can be plug-in fitted with the tank body 1, so that the two portions can be mounted in the tank body 1 or removed from the tank body 1 as a whole. In this way, the air duct assembly in the humidification tank can be mounted or removed as an independent module, which reduces the difficulty of processing and assembling for a higher assembling efficiency. In addition, this structure is also convenient for users to clean the intake airflow guide portion 2 and the outtake airflow guide portion 3. Moreover, after the intake airflow guide portion 2 and the outtake airflow guide portion 3 are removed, the interior of the tank body 1 is a complete cavity, thereby increasing the volume of the internal chamber 13, so that users can put in more humidification liquid 4, for a higher humidification efficiency.

Further, as shown in FIG. 1 and FIG. 3, the airflow inlet 16 and the airflow outlet 17 are located on the same side of the tank body 1, which further facilitates pipeline connecting and optimizes the pipeline layout.

Optionally, as shown in FIG. 1, FIG. 18, and FIG. 23, the intake airflow guide portion 2 or the outtake airflow guide portion 3 is provided with a positioning structure, and the upper cover 12 is provided with a matching structure matched with the positioning structure.

When the intake airflow guide portion 2 and the outtake airflow guide portion 3 are mounted, the positioning structure and the matching structure can be taken as the reference for the convenience of mounting. In addition, this design can make the structure have a foolproof feature, so that users can mount the intake airflow guide portion 2 and the outtake airflow guide portion 3 to the upper cover 12 in correct postures, thereby reducing the difficulty of assembling and improving the convenience of use.

Further, as shown in FIG. 1 and FIG. 23, the matching structure is a rib 121 provided on the upper cover 12, and the positioning structure is a notch 24 provided corresponding to the rib 121. They have identical or similar shapes, so that users can directly know the corresponding relationship between them. The positioning structure may be a rib, and correspondingly, the matching structure will be a notch. Alternatively, the positioning structure and the matching structure may be other structures respectively, as long as they can limit the positions of the intake airflow guide portion 2 and the outtake airflow guide portion 3 for mounting. Therefore, there is no limit to their structures herein.

Optionally, as shown in FIG. 2, the bottom shell 11 is further provided with a heat-conducting sheet 15, which is sealed and fixed to the bottom shell 11 to transfer the heat of the heating device to the internal chamber 13, so that the airflow in the internal chamber 13 is not only humidified but also warmed up, making the user's breathing more comfortable and improving the use experience.

The flowing path of the airflow of the ventilator when it passes the humidification tank according to the present invention is as follows: The airflow sent by a blower enters the first end 21 of the intake airflow guide portion 2 through the airflow inlet 16; then it enters the flow guide pipe 23, and makes multiple turns inside the flow guide pipe 23, passing the first flow channel 231 and the second flow channel 232 in sequence; then it is rectified by the flow rectification portion 25 at the second end 22, and flows obliquely downward under the guidance of the liquid damper 26 to enter the internal chamber 13, where it contacts the humidification liquid 4 below and gets moistened; then the moistened airflow enters the outtake airflow guide portion 3 through the third end 31, and flows out from the fourth end 32 through the airflow outlet 17; and finally the airflow flows to the user end through the breathing tube and the mask, and is used by the user.

Any feature not covered in the present invention can be realized by adopting or referring to the prior art.

The embodiments in the specification are described in a progressive manner. The same or similar parts between the embodiments can be referred to each other. Each embodiment focuses on the differences from other embodiments.

The above descriptions are only embodiments of the present invention and are not intended to limit the present invention. Various changes and variations of the present invention are possible for those skilled in the art. Any modifications, equivalent substitutions, improvements, etc., made within the spirit and principles of the present invention shall be included within the scope of the claims of the present invention.

## Claims

1. A humidification tank for a ventilator, comprising a tank body and an internal chamber, wherein the tank body is provided with an airflow inlet and an airflow outlet, and the internal chamber is configured to contain a humidification liquid and to be in communication with an external air path via the airflow inlet and the airflow outlet, wherein
the tank body comprises an insertion end and a graspable end, wherein the insertion end is configured to be inserted into a ventilator unit; the humidification tank further comprises an intake airflow guide portion having a first end and a second end, wherein the first end is hermetically connected to the airflow inlet, and the second end is connected to the internal chamber; and the intake airflow guide portion comprises a bent flow guide pipe having at least two bends, such that the first end and the second end are located on two opposite sides of the intake airflow guide portion; or
the intake airflow guide portion comprises a flow guide pipe that is bent and extends, and the flow guide pipe comprises a first flow channel and a second flow channel, which are interconnected with each other, wherein the internal chamber and the flow guide pipe are configured such that when the humidification tank is rotated from an operating orientation toward any direction by an angle of 90° or less, the humidification liquid cannot enter the first flow channel, so that the humidification liquid is prevented from flowing out of the airflow inlet.

2. The humidification tank for a ventilator according to claim 1, wherein the bent flow guide pipe comprises a first section, a second section, and a third section, which are connected in sequence,the first section and the third section extend along a first direction, and the second section extends in a direction opposite to the first direction.

3. The humidification tank for a ventilator according to claim 2, wherein the first section, the second section, and the third section are arranged side by side, and the third section is positioned higher than the first section and/or the second section.

4. The humidification tank for a ventilator according to claim 3, wherein the bottom height of the third section gradually decreases along the first direction.

5. The humidification tank for a ventilator according to claim 2 or 4, wherein the third section is provided with an anti-backflow rib protruding upward on bottom wall of the third section.

6. The humidification tank for a ventilator according to claim 2, wherein the change in cross-sectional area between the first section and the second section and/or the change in cross-sectional area between the second section and the third section is less than 10%.

7. The humidification tank for a ventilator according to claim 2, wherein the width of the third section gradually increases along the first direction.

8. The humidification tank for a ventilator according to claim 2, wherein the intake airflow guide portion further comprises a flow rectification portion disposed at the second end, wherein an inlet of the flow rectification portion is connected to an outlet of the intake airflow guide portion, an outlet of the flow rectification portion is connected to the internal chamber, and the outlet of the flow rectification portion is positioned lower than the inlet of the flow rectification portion.

9. The humidification tank for a ventilator according to claim 8, wherein the outlet of the flow rectification portion is positioned lower than the first section.

10. The humidification tank for a ventilator according to claim 8, wherein the outlet of the flow rectification portion is configured such that airflow enters the internal chamber in a direction different from the first direction.

11. The humidification tank for a ventilator according to claim 8, wherein a liquid damper is provided on the lower side of the outlet of the flow rectification portion, and the liquid damper extends toward the first end and to a position below the intake airflow guide portion.

12. The humidification tank for a ventilator according to claim 11, wherein the liquid damper extends obliquely downward to form a flow guide slope on the surface of the liquid damper.

13. The humidification tank for a ventilator according to claim 8, wherein the flow rectification portion is provided with a flow guide rib, which is bent and extends to guide the airflow to flow toward the outlet of the flow rectification portion.

14. The humidification tank for a ventilator according to claim 1, wherein the humidification tank further comprises an outtake airflow guide portion having a third end and a fourth end, wherein the third end is in communication with the internal chamber, and the fourth end is hermetically connected to the airflow outlet.

15. The humidification tank for a ventilator according to claim 14, wherein the humidification tank comprises a base and a cover configured to be snap-fitted with each other, and the airflow inlet and the airflow outlet are disposed on the cover and extend toward the internal chamber to form an airflow inlet mounting portion and an airflow outlet mounting portion, respectively.

16. The humidification tank for a ventilator according to claim 15, wherein the intake airflow guide portion and the outtake airflow guide portion are configured as an integrated structure, and can be integrally plug-in fitted with the airflow inlet mounting portion and the airflow outlet mounting portion, respectively.

17. The humidification tank for a ventilator according to claim 16, wherein the intake airflow guide portion or the outtake airflow guide portion is provided with a positioning structure, and the cover is provided with a matching structure matched with the positioning structure.

18. The humidification tank for a ventilator according to claim 1, wherein the airflow inlet is disposed on the front side of the tank body, both the first flow channel and the second flow channel extend along the front-to-back direction of the tank body, and when the humidification tank is rotated forward from the operating orientation by an angle of 90° or less, the second end is higher than the level of the humidification liquid.

19. The humidification tank for a ventilator according to claim 18, wherein the flow guide pipe is provided inside with a partition, which is located between the first flow channel and the second flow channel, and when the humidification tank is rotated backward from the operating orientation by an angle of 90° or less, the highest point of the partition is higher than the level of the humidification liquid.

20. The humidification tank for a ventilator according to claim 1, wherein the airflow inlet is disposed on the front side of the tank body, the flow guide pipe is provided inside with a partition, which is located between the first flow channel and the second flow channel, and when the humidification tank is rotated leftward or rightward from the operating orientation by an angle of 90° or less, at least a part of the partition is higher than the level of the humidification liquid.

21. The humidification tank for a ventilator according to claim 1, wherein the intake airflow guide portion further comprises a flow rectification portion disposed at the second end thereof, wherein an inlet of the flow rectification portion is connected to an outlet of the intake airflow guide portion, the outlet of the flow rectification portion is connected to the internal chamber, and the position of the outlet of the flow rectification portion is lower than the position of the inlet of the flow rectification portion.

22. The humidification tank for a ventilator according to claim 21, wherein a liquid damper is provided on the lower side of the outlet of the flow rectification portion, and when the humidification tank is in the operating orientation, the lowest end of the liquid damper is higher than the level of the humidification liquid.

23. The humidification tank for a ventilator according to claim 22, wherein the liquid damper extends obliquely downward along the flow rectification portion and to a position below the intake airflow guide portion.

24. The humidification tank for a ventilator according to claim 21, wherein the flow rectification portion is provided inside with a flow guide rib, which is bent and extends to guide the airflow to flow toward the outlet of the flow rectification portion.

25. The humidification tank for a ventilator according to claim 1, wherein the tank body comprises a bottom shell and an upper cover, wherein the bottom shell and the upper cover enclose the internal chamber, the intake airflow guide portion is disposed on the upper cover, and when the humidification tank is rotated from the operating orientation by 180°, the first end or the second end is higher than the level of the humidification liquid.

26. The humidification tank for a ventilator according to claim 14, wherein the first end is provided with an air inlet, and the fourth end is provided with a humidification outlet, wherein the air inlet and the humidification outlet face the same side, the airflow inlet and the air inlet are arranged correspondingly, and the airflow outlet and the humidification outlet are arranged correspondingly.
